# EUROPÄISCHE PATENTSCHRIFT

(11) **EP 1 133 486 B1**
(45) Veröffentlichungstag und Bekanntmachung des Hinweises auf die Patenterteilung: **22.12.2004**
(21) Anmeldenummer: 99963476.9
(22) Anmeldetag: 29.11.1999
(51) Int. Cl.: C07D 307/88, C07D 265/02, A61K 31/16, A61K 31/365, A61K 31/38, A61K 31/535, A61K 31/50, A61P 23/00, A61K 31/165, A61P 29/00

(54) **NICHTSTEROIDALE ENTZÜNDUNGSHEMMER**
NONSTEROIDAL ANTIINFLAMMATORIES
INHIBITEURS D'INFLAMMATION NON STEROIDIQUES

(30) Priorität: 27.11.1998 DE 19856475
(43) Veröffentlichungstag der Anmeldung: 19.09.2001
(73) Patentinhaber: SCHERING AKTIENGESELLSCHAFT, 13342 Berlin (DE)
(72) Erfinder: LEHMANN, Manfred, D-12305 Berlin (DE); KROLIKIEWICZ, Konrad, D-12357 Berlin (DE); SKUBALLA, Werner, D-13465 Berlin (DE); STREHLKE, Peter, D-10629 Berlin (DE); KALKBRENNER, Frank, D-10713 Berlin (DE); EKERDT, Roland, D-13469 Berlin (DE); GIESEN, Claudia, D-13587 Berlin (DE)
(86) Internationale Anmeldenummer: PCT/EP1999/009754
(87) Internationale Veröffentlichungsnummer: WO 2000/032584

(56) Entgegenhaltungen:
- EP-A- 0 173 516
- EP-A- 0 253 500
- EP-A- 0 253 503
- WO-A-98/54159

## Beschreibung

Die vorliegende Erfindung betrifft die Verwendung nicht - steroidaler Verbindungen zur Herstellung von Arzneimitteln die eine entzündungshemmende Aktivität aufweisen sowie nichtsteroidale Verbindungen selbst

Neben einer großen Zahl von Steroidverbindungen, die gut am Glucocorticoid - Rezeptor binden und entzündungshemmend wirken (Glucocorticoide), sind nichtsteroidale Verbindungen bekannt, die zwar am Glucocorticoid - Rezeptor binden, für die bisher aber keine Entzündungshemmung gezeigt wurde [vgl. Nature Medicin 4 (1998) 92, Mol. Pharmacol. **52** (1997) 571]. Des weiteren wurden nichtsteroidale Verbindungen beschrieben, die sich von steroidalen Verbindungen ableiten, Affinität zum Glucocorticoid - Rezeptor aufweisen und wahrscheinlich rezeptorvermittelt anti - inflammatorisch wirksam sind [J. Med. Chem. 36, 3278-3285]. Diese Verbindungen zeigten in den Tierexperimenten allerdings keine Vorteile gegenüber steroidalen Glucocorticoiden, d.h. es war nicht möglich, die anti - inflammatorische Wirkung von metabolischen Effekten, z.B. Suppression der Nebennierenfunktion, zu trennen.

Es wurden nun nichtsteroidale Verbindungen gefunden, die gut an den Glucocorticoid - Rezeptor binden und, vermittelt über diese Bindung, eine Entzündungshemmung bewirken. Diese Verbindungen zeigen im Tierexperiment deutliche Dissoziationen zwischen anti - inflammatorischen und metabolischen Wirkungen und sind daher sowohl den bisher beschrieben steroidalen, als auch den nichtsteroidalen Glucocorticoiden überlegen.

Gemäß der vorliegenden Erfindung geeignete Verbindungen zur Herstellung von Arzneimitteln, die eine entzündungshemmende Wirkung aufweisen, sind die nichtsteroidalen Verbindungen der allgemeinen Formel I 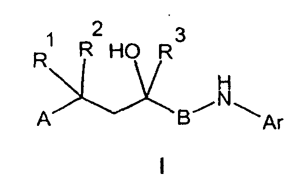 worin
- R¹ und R²: Gleich oder verschieden sind und für ein Wasserstoffatom, eine C₁-C₅-Alkylgruppe oder, gemeinsam mit dem C-Atom der Kette, für einen Ring mit insgesamt 3-7 Gliedern,
- R³: für eine C₁-C₅ Alkylgruppe oder eine teilweise oder vollständig fluorierte C₁-C₅ Alkylgruppe,
- A: für die Gruppe 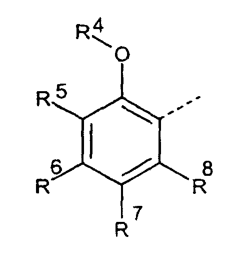 (die unterbrochene Linie bedeutet die Verknüpfungsstelle), worin R⁴ ein Wasserstoffatom, eine C₁-C₅-Alkylgruppe, eine C₁-C₁₀-Acylgruppe, eine C₃-C₁₀-Carbalkoxyalkylgruppe, eine C₂-C₅-Cyanalkylgruppe, eine C₃-C₁₀ unsubstituierte oder substituierte Allylgruppe, eine C₃-C₁₀ unsubstituierte oder substituierte Propargylgruppe, eine C₂-C₅-Alkoxyalkylgruppe, eine teilweise oder vollständig durch Fluoratome substituierte C₁-C₅-Alkylgruppe , R⁵ bis R⁸ gleich oder verschieden von einander und ausgewählt sind aus Wasserstoff- oder Halogenatomen oder C₁-C₅ Alkoxygruppen,
sowie R⁴ und R⁵ gemeinsam einen heterocyclischen Ring, der zusätzlich zum Sauerstoffatom gegebenenfalls mindestens ein weiteres Heteroatom aus der Gruppe Sauerstoff, Stickstoff, Schwefel enthalten kann, mit insgesamt 5-7 Gliedern bedeuten,
- B: für eine Carbonyl- oder eine CH₂-Gruppe und
- Ar: für ein Ringsystem, ausgewählt aus der Gruppe der allgemeinen Teilformeln 2 - 5, stehen, 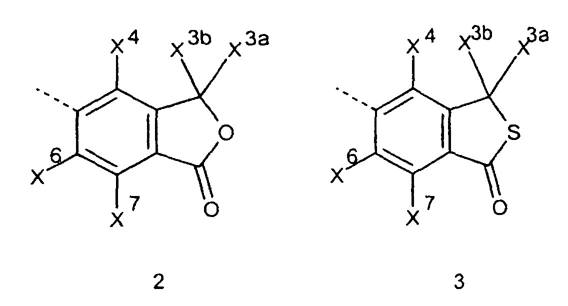 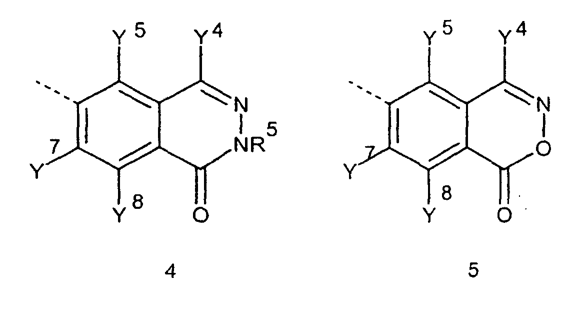
worin
die Reste X^{3a}, X^{3b}, X⁴, X⁶, X⁷ (in den Teilformeln **2** und **3**) und Y⁴, Y⁵, Y⁷, Y⁸ (in den Teilformeln **4** und **5)** gleich oder verschieden und ausgewählt sind aus Wasserstoffatomen, C₁-C₅-Alkylgruppen, teilweise oder vollständig fluorierten C₁-C₅ Alkylgruppen,
sowie darüber hinaus die Reste X⁴, X⁶, X⁷ (in den Teilformeln **2** und **3)** oder Y5, Y⁷, Y⁸ (in den Teilformeln **4** und **5)** ausgewählt sind aus Halogenatomen, Hydroxygruppen, C₁-C₅-Alkoxygruppen oder C₁-C₅-Alkanoyloxygruppen, sowie für den Fall, daß B für eine CH₂-Gruppe steht, die physiologisch verträglichen Salze der Verbindungen der allgemeinen Formel I mit Säuren.

Die erfindungsgemäßen Verbindungen der allgemeinen Formel I können durch das Vorhandensein von Asymmetriezentren als unterschiedliche Stereoisomere vorliegen. Sowohl die Racemate als auch die getrennt vorliegenden Stereoisomere gehören zum Gegenstand der vorliegenden Erfindung.

Die als Gruppen definierten Substituenten in den Verbindungen der allgemeinen Formel I können jeweils die nachfolgenden Bedeutungen haben.

Bei den C₁-C₅-Alkylgruppen kann es sich durchweg um eine Methyl-, Ethyl-, n-Propyl-, iso-Propyl-, n-, iso-, tert.-Butyl- oder um eine n-Pentyl-, 2,2-Dimethylpropyl- oder 3-Methylbutylgruppe handeln. Eine Methyl- oder Ethylgruppe ist bevorzugt.

Für ein Halogenatom kann ein Fluor-, Chlor-, Brom- oder lodatom stehen. Bevorzugt ist hier Fluor, Chlor oder Brom.

Wenn R¹ und R² gemeinsam mit dem C-Atom der Kette einen 3-7 gliedrigen Ring bilden, so ist dies beispielsweise ein Cyclo-propyl-, -butyl-, -pentyl- oder -hexylring. Der Cyclopropylring ist bevorzugt.

Für eine teilweise oder vollständig fluorierte C₁-C₅-Alkylgruppe kommen die perfluorierten, oben stehenden Alkylgruppen, und von diesen vor allem die Trifluormethyl- oder Pentafluorethylgruppe sowie als teilweise fluorierte Alkylgruppen beispielsweise die 5,5,5,4,4-Pentafluorpentyl- oder 5,5,5,4,4,3,3-Heptafluorpentylgruppe in Betracht.

Für die C₃-C₁₀- Carbalkoxyalkylgruppe kann beispielsweise eine Carboxymethyl-, tert.-Butoxymethyl- oder Ethoxymethylgruppe stehen; die beiden zuerst genannten sind bevorzugt. Die Angabe der C-Atome bezieht sich auf die insgesamt in der Carbalkoxyalkylgruppe enthaltenen C-Atome.

Als Vertreter für die C₂-C₅-Cyanalkylgruppe seien Cyanmethyl sowie 1- und 2-Cyanethyl genannt; Cyanmethyl ist bevorzugt.

Die C₃-C₁₀-Allylgruppe ist vorzugsweise eine unsubstituierte Allylgruppe; im Falle einer substituierten Allylgruppe seien beispielsweise 1-Methylallyl, 1,1-Dimethylallyl, 2-Methylallyl, 3-Methylallyl, 2,3-Dimethylallyl, 3,3-Dimethylallyl, Cinnamyl und 3-Cyclohexylallyl genannt.

Eine unsubstituierte Propargyl-, eine Methylpropargyl-, 3-Methylpropargyl-, 3-Phenylpropargyl- oder 3-Cyclohexylpropargylgruppe sind die exemplarischen Vertreter für eine C₃-C₁₀ Propargylgruppe; die unsubstituierte Propargylgruppe ist bevorzugt.

Für C₂-C₅-Alkoxyalkyl kann beispielsweise Methoxymethyl, Ethoxymethyl oder 2-Methoxyethyl stehen.

Vertreter für eine C₁-C₅-Alkoxygruppe sind ausgewählt aus Methoxy-, Ethoxy-, n-Propoxy-, iso-Propoxy-, n-, iso-, tert.-Butoxy- oder n-Pentoxy-, 2,2-Dimethylpropoxyoder 3-Methylbutoxygruppen. Eine Methoxy- oder Ethoxygruppe ist bevorzugt.

C₁-C₅-Perfluoralkoxygruppen sind die entsprechenden perfluorierten Reste der vorstehenden C₁-C₅-Alkoxygruppen.

Als C₁-C₅-Alkylgruppe zur Veretherung von Hydroxygruppen kommen die genannten Alkylgruppen in Frage, in erster Linie eine Methyl- oder Ethylgruppe. Als C₁-C₅-Alkanoylgruppe zur Veresterung von Hydroxygruppen kommt eine Formyl,-Acetyl-, Propionyl-, Butyryl-, iso-Butyryl-, Valeryl- oder iso-Valeryl- oder Pivaloylgruppe in Betracht, vorzugsweise eine Acetylgruppe.
Als C₁-C₁₀-Acylgruppe zur Veresterung von Hydroxygruppen seien beispielsweise die vorstehend genannten Alkanoylgruppen, vorzugsweise wiederum eine Acetylgruppe, oder eine Benzoyl-, Toluoyl-, Phenylacetyl-, Acryloyl-, Cinnamoyl- oder Cyclohexylcarbonylgruppe genannt.

Als C₁-C₅-Alkanoyloxygruppe für X⁴, X⁶, X⁷, Y⁴, Y⁵, Y⁷ oder Y⁸ kommt eine Formyloxy,- Acetoxy-, Propionyloxy-, Butyryloxy-, iso-Butyryloxy-, Valeryloxy- oder iso-Valeryloxygruppe in Betracht, vorzugsweise eine Acetoxygruppe.

Im Falle, daß die Verbindungen der allgemeinen Formel I (B = -CH₂-) als Salze vorliegen, kann dies beispielsweise in der Form des Hydrochlorids, Sulfats, Nitrats, Maleats, Fumarats, Tartrats oder Benzoats sein.

Wenn die erfindungsgemäßen Verbindungen als racemische Gemische vorliegen, können sie nach dem Fachmann geläufigen Methoden der Racemat - Trennung in die reinen, optisch aktiven Formen aufgetrennt werden. Beispielsweise lassen sich die racemischen Gemische durch Chromatographie an einem selbst optisch aktiven Trägermaterial (CHIRALPAK AD®) in die reinen Isomere trennen. Es ist auch möglich, die freie Hydroxygruppe in einer racemischen Verbindung der allgemeinen Formel I mit einer optisch aktiven Säure zu verestern und die erhaltenen diastereoisomeren Ester durch fraktionierte Kristallisation oder chromatographisch zu trennen und die getrennten Ester jeweils zu den optisch reinen Isomeren zu verseifen. Als optisch aktive Säure kann beispielsweise Mandelsäure, Camphersulfonsäure oder Weinsäure verwendet werden.

Bevorzugt gemäß vorliegender Erfindung sind solche Verbindungen der allgemeinen Formel 1, in denen:
- R¹ und R²: gleich oder verschieden sind und für ein Wasserstoffatom, eine Methyl- oder Ethylgruppe, ferner gemeinsam mit dem C-Atom der Kette für einen Cyclopropylring stehen, und/oder
- R³: für eine C₁-C₅ Perfluoralkylgruppe, und/oder
- A: die Gruppe 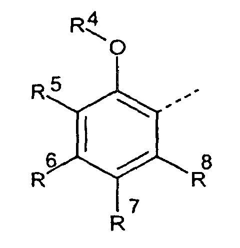 (die unterbrochene Linie bedeutet die Verknüpfungsstelle)
worin
R⁴ ein Wasserstoffatom, eine Methyl-, Ethyl-, Propyl- oder 2-Propylgruppe, eine Acetylgruppe, eine Methoxy-, Ethoxy- oder tert.Butoxycarbonylgruppe, eine Cyanmethyl-, 2-Cyanethylgruppe, eine Allylgruppe, eine Propargylgruppe, eine Methoxymethyl-, Methoxyethyl- oder Ethoxyethylgruppe, eine Mono-, Di- oder Trifluormethylgruppe, eine Pentafluorethyl- oder Nonafluorbutylgruppe,
R⁵ bis R⁸ in ein oder zwei Positionen Fluor- oder Chloratome und in den restlichen Positionen Wasserstoffatome bedeuten,
oder
R⁴ und R⁵ gemeinsam unter Einbeziehurig der Phenyl-Ringatome 2 und 3 einen Furan-, einen Dihydrofuran- oder einen 2,3-Dihydro-1,4-dioxinring und R⁶, R⁷ und R⁸ Wasserstoffatome bedeuten,
- X^{3a}: für ein Wasserstoffatom oder eine Methylgruppe, oder
- X^{3a} und X^{3b}: gleich oder verschieden sind und für ein Wasserstoffatom oder eine Methylgruppe,
- X⁴, X⁶ und X⁷: gleich oder verschieden sind und unabhängig voneinander für ein Wasserstoffatom oder ein Fluor- oder ein Chloratom, und/oder
- Y⁴: für eine Methyl- Ethyl-, Propyl-, 2-Propyl- oder Trifluormethylgruppe und/oder
- Y⁵, Y⁷ und Y⁸: gleich oder verschieden sind und unabhängig voneinander für ein Wasserstoffatom oder ein Fluor- oder ein Chloratom
stehen,
und die anderen Substituenten alle in der Formel I angegebenen Bedeutungen haben.

Des weiteren ist die Verwendung solcher Verbindungen der allgemeinen Formel I bevorzugt, in denen Ar für ein Ringsystem der Teilformel **2** oder **5** steht.

Die Verwendung der nachstehend genannten Verbindungen ist erfindungsgemäß insbesondere bevorzugt:
5-{2-Hydroxy-3-[1-(2-methoxyphenyl)-cyclopropyl]-2-trifluormethyl-propionylamino}phthalid
5-{3-[1-(5-Fluor-2-methoxyphenyl)-cyclopropyl]-2-hydroxy-2-trifluormethyl-propionylamino}-phthalid
5-[2-Hydroxy-4-(2-methoxyphenyl)-4-methyl-2-trifluormethyl-valeroylamino]-phthalid
5-{3-[1-(5-Fluor-2-hydroxyphenyl)-cyclopropyl]-2-hydroxy-2-trifluormethyl-propionylamino}-phthalid
5-[4-(5-Fluor-2-hydroxyphenyl)-2-hydroxy-4-methyl-2-trifluormethyl-valeroylamino]-phthalid
5-[4-(5-Fluor-2-hydroxyphenyl)-2-hydroxy-4-methyl-2-trifluormethyl-pentylamino]-phthalid
4-Brom-5-[4-(5-fluor-2-hydroxyphenyl)-2-hydroxy-4-methyl-2-trifluormethyl-pentylamino]-phthalid
4-Brom-5-[4-(3-brom-5-fluor-2-hydroxyphenyl)-2-hydroxy-4-methyl-2-trifluormethyl-pentylamino]-phthalid
6-{3-[1-(5-Fluor-2-hydroxyphenyl)-cyclopropyl]-2-hydroxy-2-trifluormethyl-propionylamino}-4-methyl-2,3-benzoxazin-1-on
6-[4-(5-Fluor-2-methoxyphenyl)-2-hydroxy-4-methyl-2-trifluormethyl-valeroylamino]-4-methyl-2,3-benzoxazin-1-on
6-[4-(5-Fluor-2-hydroxyphenyl)-2-hydroxy-4-methyl-2-trifluormethyl-valeroylamino]-4-methyl-2,3-benzoxazin-1-on
6-[2-Hydroxy-4-(2-hydroxyphenyl)-4-methyl-2-trifluormethyl-valeroylamino]-4-ethyl-2,3-benzoxazin-1-on
6-[4-(5-Fluor-2-methoxyphenyl)-2-hydroxy-4-methyl-2-trifluormethyl-pentylamino]-4-methyl-2,3-benzoxazin-1-on
6-[4-(5-Fluor-2-hydroxyphenyl)-2-hydroxy-4-methyl-2-trifluormethyl-pentylamino]-4-methyl-2,3-benzoxazin-1-on
6-[4-(4-Brom-2-methoxyphenyl)-2-hydroxy-4-methyl-2-trifluormethyl-valeroylamino]-4-methyl-2,3-benzoxazin-1-on
6-[4-(4-Brom-2-methoxyphenyl)-2-hydroxy-4-methyl-2-trifluormethyl-pentylamino]-4-methyl-2,3-benzoxazin-1-on

Des weiteren ist die Verwendung der aus den Tabellen 3 bis 6 hervorgehenden Verbindungen bevorzugt.

Bevorzugt werden alle genannten Verbindungen in Form der optischen Antipoden oder der getrennten Diastereomere verwendet.

Nichtsteroidale Verbindungen als solche mit einem gemischten Profil aus gestagener und androgener Aktivität in unterschiedlicher Ausprägung sind bereits Gegenstand der am 02. Juni 1998 eingereichten PCT - Anmeldung PCT/EP98/03242 (Priorität: 30. Mai 1997, DE 197 23 722.3). Die gemäß vorliegender Patentanmeldung zur Herstellung von Arzneimitteln mit entzündungshemmender Wirksamkeit zu verwendenden Verbindungen der allgemeinen Formel I fallen in den Umfang der in der PCT - Anmeldung PCT/EP98/03242 enthaltenen allgemeinen Formel.
Die nachstehend namentlich aufgeführten Verbindungen der allgemeinen Formel I fallen zwar in den Umfang der in der nicht - vorveröffentlichten PCT - Anmeldung PCT/EP98/03242 enthaltenen allgemeinen Formel, sind aber dort nicht namentlich vorbeschrieben. Sie sind somit neu und erfüllen aufgrund der für sie bei nichtsteroidalen Verbindungen erstmals aufgefundenen entzündungshemmenden, von metabolischen Effekten dissoziierten Wirkung, auch das Patentierungserfordernis der erfinderischen Tätigkeit. Diese Verbindungen als solche gehören deshalb ebenfalls zum Gegenstand der vorliegenden Erfindung.
5-{3-[1-(5-Fluor-2-methoxyphenyl)-cyclopropyl]-2-hydroxy-2-trifluormethyl-propionylamino}-phthalid
5-{3-[1-(5-Fluor-2-hydroxyphenyl)-cyclopropyl]-2-hydroxy-2-trifluormethyl-propionylamino}-phthalid
4-Brom-5-[4-(5-fluor-2-hydroxyphenyl)-2-hydroxy-4-methyl-2-trifluormethyl-pentylamino]-phthalid
4-Brom-5-[4-(3-brom-5-fluor-2-hydroxyphenyl)-2-hydroxy-4-methyl-2-trifluormethyl-pentylamino]-phthalid
6-{3-[1-(5-Fluor-2-hydroxyphenyl)-cyclopropyl]-2-hydroxy-2-trifluormethyl-propionylamino}-4-methyl-2,3-benzoxazin-1-on
6-[2-Hydroxy-4-(2-hydroxyphenyl)-4-methyl-2-trifluormethyl-valeroylamino]-4-ethyl-2,3-benzoxazin-1-on
6-[4-(4-Brom-2-methoxyphenyl)-2-hydroxy-4-methyl-2-trifluormethyl-pentylamino]-4-methyl-2,3-benzoxazin-1-on
5-{3-[1-(5-Fluor-2-methoxyphenyl)-cyclopropyl]-2-hydroxy-2-trifluormethyl-propionylamino}-phthalid
6-[2-Hydroxy-4-(5-fluor-2-methoxyphenyl)-4-methyl-2-trifluomethyl-pentylamino]-4-methyl-2,3-benzoxazin-1-on
5-{3-[1-(5-Fluor-2-hydroxyphenyl)-cyclopropyl]-2-hydroxy-2-trifluormethyl-propionylamino}-phthalid
6-{3-[1-(5-Fluor-2-hydroxyphenyl)-cyclopropyl]-2-hydroxy-2-trifluormethyl-propionylamino}-4-methyl-2,3-benzoxazin-1-on
(-)-4-Brom-5-[4-(5-fluor-2-hydroxyphenyl)-2-hydroxy-4-methyl-2-trifluormethyl-pentylamino]-phthalid
(-)-4-Brom-5-[4-(3-brom-5-fluor-2-hydroxyphenyl)-2-hydroxy-4-methyl-2-trifluormethyl-pentylamino]-phthalid
5-[2-Hydroxy-4-(5-isopropyl-2-methoxyphenyl)-4-methyl-2-trifluormethyl-valeroylamino]-phthalid
5-[2-Hydroxy-4-(2-methoxy-5-propyl-phenyl)-4-methyl-2-trifluormethyl-valeroylamino]-phthalid
5-[2-Hydroxy-4-(2-benzyloxy-5-fluorphenyl)-4-methyl-2-trifluormethyl-valeroylamino]-phthalid
5-[2-Hydroxy-4-(2-difluormethoxy-5-fluorphenyl)-4-methyl-2-trifluormethyl-valeroylamino]-phthalid
5-[2-Hydroxy-4-(5-fluor-2-methoxymethoxy-phenyl)-4-methyl-2-trifluormethyl-valeroylamino]-phthalid
5-[2-Hydroxy-4-(2-ethoxymethoxy-5-fluorphenyl)-4-methyl-2-trifluormethyl-valeroylamino]-phthalid
5-{[2-Hydroxy-4-[5-fluor-2-(2-methoxyethoxy)-phenyl]-4-methyl-2-trifluormethyl-valeroylamino}-phthalid

### Pharmakologische Daten

Im Glucocorticoid - Rezeptor (GR) - Bindungstest unter Verwendung von Cytosolpräparationen aus Thymushomogenaten der Ratte und von [³H]-Dexamethason als Bezugssubstanz zeigen die Verbindungen der Formel I eine hohe bis sehr hohe Affinität zum GR.

Zusätzlich zeigen diese Verbindungen im Gestagen - Rezeptor - Bindungstest unter Verwendung von Cytosolpräparationen aus Kaninchenuterushomogenaten und von [³H] - Progesteron als Bezugssubstanz Affinitäten zum Gestagen - Rezeptor.

Des weiteren zeigen diese Verbindungen im Mineralcorticoid - Rezeptor (MR) - Bindungstest unter Verwendung von Cytosolpräparationen aus Ratten Hippocampi und von [³H]-Aldosteron als Bezugssubstanz Affinitäten zum MR.

Als wesentlicher, molekularer Mechanismus für die anti - inflammatorische Wirkung von Glucocorticoiden wird die durch den GR vermittelte Hemmung der Transkription von Cytokinen, Adhäsionsmolekülen, Enzymen und anderer pro - inflammatorischen Faktoren angesehen. Diese Hemmung wird durch eine Interaktion des GR mit anderen Trankriptionsfaktoren, z.B. AP-1 und NF-kappa-B, bewirkt (zur Übersicht siehe Cato, ACB and Wade E, BioEssays **18**, 371-378 1996).

Die erfindungsgemäßen Verbindungen der allgemeinen Formel I hemmen die durch Lipopolysacchard (LPS) ausgelöste Sekretion des Cytokins IL-8 in der menschlichen Monozytenzelline THP-1. Die Konzentration der Cytokine wurde im Überstand mittels kommerziell erhältlicher ELISA-Kits bestimmt.

Die anti - inflammatorische Wirkung der Verbindungen der allgemeinen Formel I wurden im Tierexperiment durch Testen in der Crotonöl - induzierten Entzündung in der Ratte und der Maus getestet. Hierzu wurde den Tieren Crotonöl in ethanolischer Lösung topisch auf die Ohren appliziert. Die Testsubstanzen wurden gleichzeitig oder zwei Stunden vor dem Crotonöl ebenfalls topisch oder systemisch appliziert. Nach 16-24 Stunden wurden das Ohrgewicht als Maß für das entzündliche Ödem, die Peroxidaseaktivität als Maß für die Einwanderungen von Granulozyten und die Elastaseaktivität als Maß für die Einwanderung von neutrophilen Granulozyten gemessen. Die Verbindungen der allgemeinen Formel I hemmen in diesem Test sowohl nach topischer, als auch nach systemischer Applikation die drei oben genannten Entzündungsparameter.

Eine der häufigsten unerwünschten Wirkungen einer Glucocorticoid - Therapie ist der sogenannte "Steroiddiabetes" [vgl. Hatz, HJ, Glucocorticoide: Immunologische Grundlagen, Pharmakologie und Therapierichtlinien, Wissenschafliche Verlagsgesellschaft mbH, Stuttgart, 1998]. Ursache hierfür ist die Stimulation der Gluconeogenese in der Leber durch Induktion der hierfür verantwortlichen Enzyme und durch freie Aminosäuren, die aus dem Abbau von Proteinen (katabole Wirkung der Glucocorticoide) entstehen. Ein Schlüsselenzym des katabolen Stoffwechsels in der Leber ist die Tyrosinaminotranferase (TAT). Die Aktivität dieses Enzyms kann photometrisch aus Leberhomogenaten bestimmt werden und stellt ein gutes Maß für die unerwünschten metabolischen Wirkungen der Glucocorticoide dar. Zur Messung der TAT - Induktion werden die Tiere 8 Stunden nach Gabe der Testsubstanzen getötet, die Leber entnommen und die TAT - Aktivität im Homogenat gemessen. Die Verbindungen der allgemeinen Formel I induzieren in diesem Test in Dosen, in denen sie anti - inflammatorisch wirksam sind, nicht oder nur in geringem Maße die Tyrosinaminotransferase.

Da die Substanzen der allgemeinen Formel auch hohe Affinität am Progesteron - Rezeptor aufweisen, wurden die neuen Verbindungen auf ihre gestagene Wirkung im Tierexperiment getestet. Hierzu wurde der Schwangerschafts - Erhaltungstest an ovariektomierten Ratten durchgeführt. Hierzu werden weibliche Ratten gedeckt, am Tag acht der Gravidität 2 Stunden nach Substanzapplikation unter Narkose ovariektomiert. An den Tagen 8 bis 14 der Schwangerschaft werden die Tiere täglich mit den Testsubstanzen behandelt und am Tag 15 werden die Tiere getötet und pro Tier die Zahl der lebenden und toten Föten ermittelt. Bei leeren Uteri wird die Zahl der Implantationsstellen durch Anfärben mit 10% Ammoniumsulfid - Lösung ermittelt. Die neuen Verbindungen der Formel I führten bis zu einer Dosis von 500 µg pro kg Körpergewicht nicht oder nur in geringem Ausmaß zur Schwangerschaftserhaltung. Wenn die tägliche Dosis auf etwa 10 mg pro kg Körpergewicht erhöht wird, ist eine abgeschwächte gestagene Wirkung zu beobachten. Wegen dieser Wirkung sind die Substanzen als Gestagene mit typischem Wirkungsspektrum nicht einsetzbar.

Zusammenfassend zeigen die neuen Verbindungen der allgemeinen Formel I gegenüber den bisherig verwendeten steroidalen Glucocorticoiden folgende Eigenschaften:
- nichtsteroidale Struktur (d.h. die Substanzen sind noch wirksam bei Patienten, die aufgrund einer allergischen Reaktion gegen die Steroidgrundstrukturen herkömmlicher Glucocorticoide für die Therpie mit diesen nicht mehr zugänglich sind (vgl. Lutz, ME, el-Azhary RA, Mayo Clin. Proc. 72, 1141-1144, 1997).
- ähnlich gute anti - inflammatorische Wirkung bei geringer metabolischer Wirkung
- schwache gestagene Wirkung, trotz der hohen Affinität zum Progesteron - Rezeptor

### Indikationen

Aufgrund ihrer anti - inflammatorischen und zusätzlichen anti - allergischen, immunsuppressiven und anti - proliferativen Wirkung können die erfindungsgemäßen Verbindungen der allgemeinen Formel I als Medikamente zur Behandlung oder Prophylaxe folgender Krankheitszustände bei Säugetieren und Menschen Verwendung finden: Dabei steht der Begriff "ERKRANKUNG" für die folgenden Indikationen:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Alle Formen der restriktiven Lungenerkrankungen, vor allem allergische Alveolitis,
   - Alle Formen des Lungenödems, vor allem toxisches Lungenödem
   - Sarkoidosen und Granulomatosen, insbesondere Morbus Boeck
(ii) Rheumatische Erkrankungen / Autoimmunerkrankungen / Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica
   - Reaktive Arthritis
   - Entzündliche Weichteilerkrankungen sonstiger Genese
   - Arthritische Symtome bei degenerativen Gelenkerkrankungen (Arthrosen)
   - Traumatische Arthritiden
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
(iii) Allergien, die mit entzündlichen, und / oder proliferativen Prozessen einhergehen:
   - Alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, Kontakdermatitis
(iv) Gefäßentzündungen (Vaskulitiden)
   - Panarteriitis nodosa, Arteriitis temporalis, Erythema nodosum
(v) Dermatologische Erkrankungen, die mit entzündlichen, allergischen und / oder proliferativen Prozessen einhergehen:
   - Atopische Dermatitis (vor allem bei Kindern)
   - Psoriasis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen
   - Erkrankungen des lichenoiden Formenkreises,
   - Pruritus (z. B. allergischer Genese)
   - Seborrhoisches Ekzem
   - Rosacea
   - Pemphigus vulgaris
   - Erythema exsudativum multiforme
   - Balanitis
   - Vulvitis
   - Haarausfall wie Alopecia areata
   - Cutane T - Zell - Lymphome
(vi) Nierenerkrankungen, die mit entzündlichen, allergischen und / oder proliferativen Prozessen einhergehen:
   - Nephrotisches Syndrom
   - Alle Nephritiden
(vii) Lebererkrankungen, die mit entzündlichen, allergischen und / oder proliferativen Prozessen einhergehen:
   - akuter Leberzellzerfall
   - akute Hepatitis unterschiedlicher Genese, z.B. viral, toxisch, arzneimittelinduziert
   - chronisch aggressive und / oder chronisch intermittierende Hepatitis
(viii) Gastrointestinale Erkrankungen, die mit entzündlichen, allergischen und / oder proliferativen Prozessen einhergehen:
   - regionale Enteritis (Morbus Crohn)
   - Colitis Ulcerosa
   - Gastritis
   - Refluxoesophagitis
   - Gastroenteritiden anderer Genese, z.B. einheimische Sprue
(ix) Proktologische Erkrankungen, die mit entzündlichen, allergischen und / oder proliferativen Prozessen einhergehen:
   - Analekzem
   - Fissuren
   - Hämorrhoiden
   - idiopathische Proktitis
(x) Augenerkrankungen, die mit entzündlichen, allergischen und / oder proliferativen Prozessen einhergehen:
   - allergische Keratitis, Uveitis, Iritis,
   - Konjunktivitis
   - Blepharitis
   - Neuritis nervi optici
   - Chorioditis
   - Ophtalmia sympathica
(xi) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis externa, z.B. bedingt durch Kontaktexem, Infektion etc.
   - Otitis media
(xii) Neurologische Erkrankungen, die mit entzündlichen, allergischen und / oder proliferativen Prozessen einhergehen:
   - Hirnödem, vor allem Tumor-bedingtes Hirnödem
   - Multiple Sklerose
   - akute Encephalomyelitis
   - Meningitis
   - verschieden Formen von Krampfanfällen, z.B. BNS-Krämpfe
(xiii) Bluterkrankungen, die mit entzündlichen, allergischen und / oder proliferativen Prozessen einhergehen:
   - Erworbene hämolytische Anämie
   - Idopathische Thrombocytopenia
(xiv) Tumorerkrankungen, die mit entzündlichen, allergischen und / oder proliferativen Prozessen einhergehen:
   - Akute lymphatische Leukämie
   - Maligne Lymphome
   - Lymphogranulomatosen
   - Lymphosarkome
   - Ausgedehnte Metastasierungen, vor allem bei Mamma- Bronchial- und Prostatakarzinom
(xv) Endokrine Erkrankungen, die mit entzündlichen, allergischen und / oder proliferativen Prozessen einhergehen:
   - Endokrine Orbitopathie
   - Thyreotoxische Krise
   - Thyreoiditis de Quervain
   - Hashimoto Thyreoiditis
   - Morbus Basedow
(xvi) Organ- und Gewebstransplantationen , Graft-versus-host-disease(xvii) Schwere Schockzustände, z.B anaphylaktischer Schock , systemic inflammatory response syndrome (SIRS)
(xviii) Substitutionstherapie bei:
   - angeborene primäre Nebenniereninsuffizienz, z.B. kongenitales adrenogenitales Syndrom
   - erworbene primäre Nebenniereninsuffizienz, z.B. Morbus Addison, autoimmune Adrenalitits, postinfektiös, Tumoren, Metastasen etc.
   - angeboren sekundäre Nebeniereninsuffizienz, z.B. kongenitaler Hypopitutitarismus
   - erworbene sekundäre Nebenniereninsuffizienz, z.B. postinfektiös, Tumoren etc.
(xix) Emesis, die mit entzündlichen, allergischen und / oder proliferativen Prozessen einhergehen:
   - z.B. in Kombination mit einem 5-HT₃-Antagonisten bei Zytostika - bedingten Erbrechen.
(xx) Schmerzen bei entzündlicher Genese, z.B. Lumbago

Darüber hinaus können die erfindungsgemäßen Verbindungen der allgemeinen Formel I zur Therapie und Prophylaxe weiterer oben nicht genannter Krankheitszustände eingesetzt werden, für die heute synthetische Glucocorticoide verwendet werden (siehe dazu Hatz, HJ, Glucocorticoide: Immunologische Grundlagen, Pharmakologie und Therapierichtlinien, Wissenschafliche Verlagsgesellschaft mbH, Stuttgart, 1998).

Alle zuvor genannten Indikationen (i) bis (xx) sind ausführlich beschrieben in Hatz, HJ, Glucocorticoide: Immunologische Grundlagen, Pharmakologie und Therapierichtlinien, Wissenschafliche Verlagsgesellschaft mbH, Stuttgart, 1998.

Für die therapeutische Wirkungen bei den oben genannten Krankheitszuständen ist die geeignete Dosis unterschiedlich und hängt beispielsweise von der Wirkstärke der Verbindung der allgemeinen Formel I, dem Wirt, der Art der Verabreichung und der Art und der Schwere der zu behandelnden Zustände, sowie der Verwendung als Prophylaktikum oder Therapeutikum ab.

Die Erfindung liefert weiterhin
(i) die Verwendung eines der erfindungsgemäßen Verbindung gemäß Formel I oder deren Gemisch zur Herstellung eines Medikaments zur Behandlung von einer ERKRANKUNG;
(ii) ein Verfahren zur Behandlung von einer ERKRANKUNG, welches Verfahren eine Verabreichung einer Verbindungsmenge gemäß der Erfindung umfaßt, wobei die Menge die Krankheit unterdrückt, und wobei die Verbindungsmenge einem Patienten gegeben wird, der ein solches Medikament benötigt;
(iii) eine pharmazeutische Zusammensetzung zur Behandlung von einer ERKRANKUNG, welche Behandlung eines der erfindungsgemäßen Verbindungen oder deren Gemisch und wenigstens einen pharmazeutischen Hilfs- und/oder Trägerstoff umfaßt.

Im allgemeinen sind bei Tieren zufriedenstellende Resultate zu erwarten, wenn die täglichen Dosen einen Bereich von 1 µg bis 100.000 µg der erfindungsgemäßen Verbindung pro kg Körpergewicht umfassen. Bei größeren Säugetieren, beispielsweise dem Menschen, liegt eine empfohlene tägliche Dosis im Bereich von 1 µg bis 100.000 µg pro kg Körpergewicht. Bevorzugt ist eine Dosis von 10 bis 30.000 µg pro kg Körpergewicht, mehr bevorzugt eine Dosis von 10 bis 10.000 µg pro kg Körpergewicht. Zum Beispiel wird diese Dosis zweckmäßigerweise mehrmals täglich verabreicht. Zur Behandlung eines akuten Schocks (z.B. anaphylaktischer Schock) können Einzeldosen gegeben werden, die deutlich über den oben genannten Dosen liegen.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw., verarbeitet und in die gewünschte Applikationsform überführt. Dabei ist auf Remington's Pharmaceutical Science, 15^{th} ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage.

Für die parenterale Applikation sind Injektion- und Infusionszubereitungen möglich.

Für die intraartikulären Injektion können entsprechend zubereitet Kristallsuspensionen verwendet werden.

Für die intramuskuläre Injektion können wässrige und ölige Injektionslösungen oder Suspensionen und entprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Aerosolen und Inhalaten verwendet werden.

Für die lokale Anwendung an Augen, äußerem Gehörgang, Mittelohr, Nasenhöhle und Nasennebenhöhlen können die neuen Verbindungen als Tropfen, Salben und Tinkturen in entsprechenden pharmazeutischen Zubereitungen verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0.01% - 20% betragen, um eine ausreichende pharmakologische Wirkung zu erzielen.

Die Erfindung umfaßt ebenfalls die erfindungsgemäßen Verbindungen der allgemeinen Formel I als therapeutischen Wirkstoff. Weiterhin gehört zur Erfindung die erfindungsgemäßen Verbindungen der allgemeinen Formel I als therapeutischen Wirkstoff zusammen mit pharmazeutisch verträglichen und annehmbaren Hilfsstoffen und Trägerstoffen. Ebenfalls umfaßt die Erfindung eine pharmazeutische Zusammensetzung, die eines der pharmazeutisch aktiven, erfindungsgemäßen Verbindungen oder deren Gemisch und ein pharmazeutisch verträgliches Salz oder pharmazeutisch verträgliche Hilfsstoffe und Trägerstoffe enthält.

Insbesondere zeigen folgende Verbindungen eine besonders effektive pharmazeutische Wirkung. Dabei ist die zweite Verbindung die bevorzugte in der Gruppe.
5-[4-(5-Fluor-2-hydroxyphenyl)-2-hydroxy-4-methyl-2-trifluormethyl-valeroylamino]-phthalid
5-[4-(5-Fluor-2-hydroxyphenyl)-2-hydroxy-4-methyl-2-trifluormethyl-pentylamino]-phthalid
6-[4-(5-Fluor-2-methoxyphenyl)-2-hydroxy-4-methyl-2-trifluormethyl-valeroylamino]-4-methyl-2,3-benzoxazin-1-on
6-[4-(4-Brom-2-methoxyphenyl)-2-hydroxy-4-methyl-2-trifluormethyl-valeroylamino]-4-methyl-2,3-benzoxazin-1-on
6-[4-(5-Fluor-2-hydroxyphenyl)-2-hydroxy-4-methyl-2-trifluormethyl-valeroylamino]-4-methyl-2,3-benzoxazin-1-on

### Beispiele:

Die erfindungsgemäßen Verbindungen der allgemeinen Formel I lassen sich wie nachstehend beschrieben herstellen:

### Herstellungsverfahren

(1). Eine Carbonylverbindung der allgemeinen Formel II 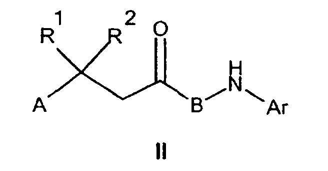 worin A, B, Ar, R¹ und R² die in Formel I angegebene Bedeutung haben, wird mit einer Verbindung der allgemeinen Formel CₙF₂ₙ₊₁-SiR³, worin R³ die in der allgemeinen Formel I angegebene Bedeutung hat, in Gegenwart eines Katalysators oder mit einer Alkylmetallverbindung, beispielsweise einem Grignard-Reagenz oder einem Lithiumalkyl, zu einer Verbindung der Formel I umgesetzt. Als Katalysator kommen Fluorid-Salze oder basische Verbindungen wie Alkalicarbonate infrage (J. Am. Chem. Soc. **111,** 393 (1989)).
(2). Eine Verbindung der allgemeinen Formel III 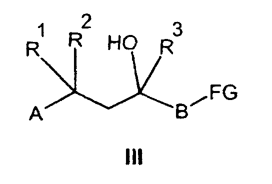 worin A,B, R¹,R² und R³ die in Formel I angegebene Bedeutung haben und FG eine Fluchtgruppe bedeutet, wird mit einer Verbindung Ar-NH-R⁹ , wobei R⁹ ein Wasserstoffatom oder eine C₁-C₅ Acylgruppe bedeutet und Ar die in der allgemeinen Formel I angegebene Bedeutung hat, umgesetzt, wobei gegebenenfalls anschließend der Rest R⁹ abgespalten wird, um zu einer Verbindung der Formel I zu gelangen. Die Verbindung der allgemeinen Formel III kann dabei gegebenenfalls nur als Zwischenprodukt gebildet werden, z.B. kann es sich um ein intermediär aus einer entsprechenden Carbonsäure gebildetes Säurechlorid handeln. Als Fluchtgruppen seien beispielsweise ein Fluor, Chlor- oder Bromatom oder der Tosylatrest genannt.
(3) Eine Verbindung der allgemeinen Formel IV 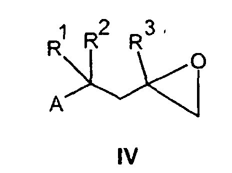 worin A, R¹,R² und R³ die in Formel I angegebene Bedeutung haben, wird mit einer Verbindung der Formel Ar-NH-R⁹, wobei R⁹ und Ar die unter 2 angegebenen Bedeutungen haben, in einem Lösemittel oder ohne ein solches umgesetzt, wobei gegebenenfalls anschließend der Rest R⁹ abgespalten wird, um zu einer Verbindung der Formel I mit B in der Bedeutung einer CH₂-Gruppe zu gelangen.
(4). Eine Verbindung der Formel I, in der R⁴ ein Wasserstoffatom bedeutet, wird mit einem geeigneten Reagenz umgesetzt, um zu Verbindungen mit Resten R⁴ in weiteren der für Formel I angegebenen Bedeutungen zu gelangen. Beispiele hierfür sind die Veretherung oder Veresterung einer entsprechenden Hydroxylverbindung.

Von den vorstehenden Verfahrensvarianten sind 1. und 2. für die Herstellung aller Verbindungen, die unter die allgemeine Formel I fallen, geeignet.

Mit der 3. Variante sind Verbindungen der allgemeinen Formel I herstellbar, worin B für eine CH₂-Gruppe steht .

Gewünschtenfalls lassen sich Verbindungen, die nach einem der vorstehenden Verfahren hergestellt wurden und in denen A ein gegebenenfalls substituierter aromatischer Ring ist, nach bekannten Verfahren an diesem aromatischen Rest selektiv substituieren. Beispiele für dieses Verfahren sind die katalytische Hydrierung von Mehrfachbindungen, die Nitrierung und die Halogenierung.

Die in den Beispielen verwendeten Ausgangsmaterialien werden folgendermaßen ergestellt:

### Herstellung der Ausgangsmaterialien

### 3-[1-(2-Methoxyphenyl)-cyclopropyl]-2-oxo-propionsäure

Entsprechend J. Org. Chem. **40** (1975) 3497 werden 16,7 g 2-Methoxyphenylacetonitril, 158 ml Lithiumtriisopropylamid (2 molare Lösung) und 46,7 ml 1,2-Dichloroethan in 96 ml Tetrahydrofuran und 58,6 ml Hexamethylphosphorsäuretriamid miteinander umgesetzt. Man erhält 5,6 g 1-(2-Methcxyphenyl)-cyclopropyl-carbonitril, Kp. 104-115°C / 0.1 mbar, die weiter wie für 3-(1-Phenyl-cyclobutyl)-2-oxo-propionsäure beschrieben, umgesetzt werden. Man erhält so 3-[1-(2-Methoxyphenyl)-cyclopropyl]-2-oxo-propionsäure als Öl.

### 4-(5-Fluor-2-methoxyphenyl)-2-hydroxy-4-methyl-2-trifluormethyl-valeriansäure

1,3 g wasserfreies Zinkchlorid und 13,2 g gekörntes Mangan werden in 100 ml Tetrahydrofuran zum Sieden erhitzt und mit 0,2 ml Methallylbromid 30 Minuten gekocht. Dann wird die Lösung von 25 g Methallylbromid und 17 g Trifluorbrenztraubensäure-ethylester in 80 ml Tetrahydrofuran im Laufe von 2 Stunden bei Siedehitze zugetropft und eine weitere Stunde gekocht. Dann gibt man unter Eiskühlung gesättigte Ammoniumchloridlösung und 300 ml Ethylacetat zu, rührt 30 Minuten bei 0°C und wäscht die abgetrennte Ethylacetat-Phase mit gesättigter Ammoniumchloridlösung und dreimal mit Wasser. Das Lösemittel wird getrocknet (Na₂SO₄) und eingedampft und der Rückstand im Vakuum destilliert. Man erhält 17,6 g 2-Hydroxy-4-methylen-2-trifluormethyl-valeriansäureethylester, Kp. 48°C / 1 hPa.

Zu 5 ml 4-Fluoranisol und 0,9 g 2-Hydroxy-4-methylen-2-trifluormethylvaleriansäure-ethylester werden 0,8 g wasserfreies Aluminiumchlorid gegeben. Nach 40 Stunden Rühren bei Raumtemperatur wird auf eiskalte 2 N Salzsäure gegeben und mit Ethylacetat extrahiert. Die Ethylacetat-Phase wird mit 1 N Salzsäure und Wasser gewaschen, getrocknet (Na₂SO₄) und eingedampft. Nach Chromatographie an Kieselgel mit Hexan / Ethylacetat (1:1) erhält man 1g 4-(5-Fluor-2-methoxyphenyl)-2-hydroxy-4-methyl-2-trifluormethyl-valeriansäureethylester, Fp. 38-39°C.

1,9 g 4-(5-Fluor-2-methoxyphenyl)-2-hydroxy-4-methyl-2-trifluormethyl-valeriansäure - ethylester werden mit 40 ml Kaliumhydroxid im Methanol (10%) 2 Stunden im Rückfluß gekocht. Nach Eindampfen des Lösemittels im Vakuum wird Wasser zugegeben, mit Hexan extrahiert und die abgetrennte Wasserphase mit 6 N Salzsäure angesäuert. Nach Extraktion mit Ethylacetat wird die Ethylacetat-Phase mit Wasser gewaschen, getrocknet (Na₂SO₄) und eingedampft. Der Rückstand wird aus Hexan kristallisiert. Man erhält 1,55 g 4-(5-Fluor-2-methoxyphenyl)-2-hydroxy-4-methyl-2-trifluormethyl-valeriansäure, Fp. 102-104 °C.

Die Säuren der Tabelle 1 wurden analog hergestellt.

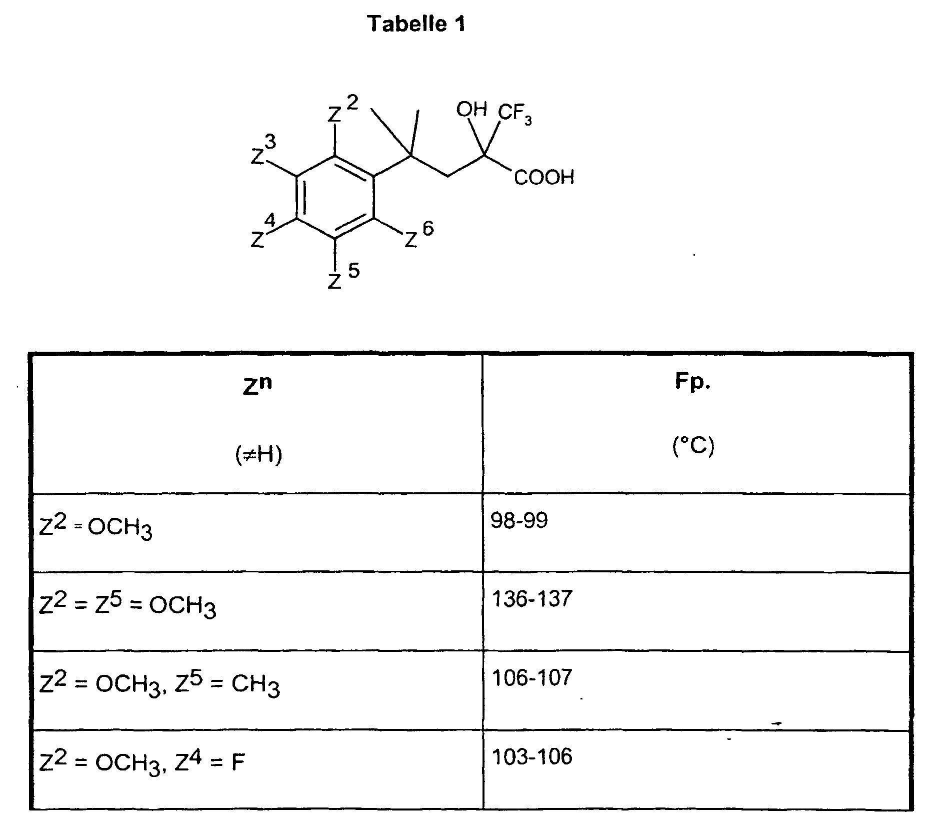

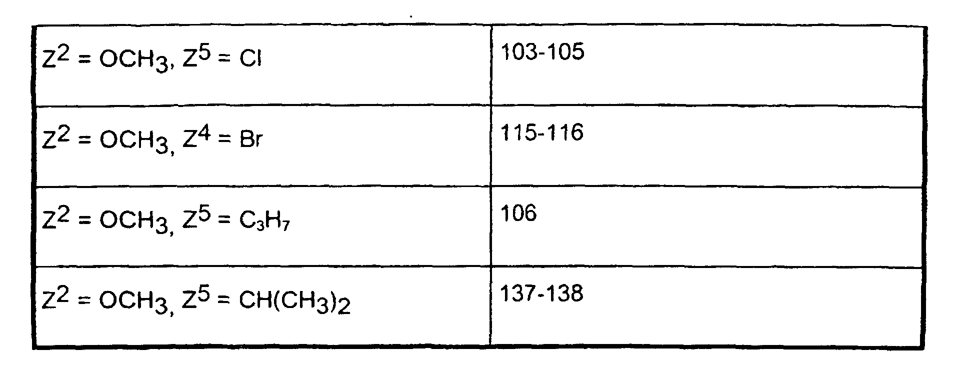

Durch Umwandlung nach Standardverfahren werden weitere Säuren aus vorstehenden Säuren oder ihren Vorstufen erhalten:

### 4-(4-Cyan-2-methoxyphenyl)-2-hydroxy-4-methyl-2-trifluormethyl-valeriansäure

Die Titelsäure wird aus 4-(4-Brom-2-methoxyphenyl)-2-hydroxy-4-methyl-2-trifluormethyl-valeriansäure - ethylester, Zinkcyanid und Tetrakis-triphenylphosphin-palladium in Dimethylformamid bei 140°C erhalten. Nach Verseifung erhält man die Titelsäure als amorphes Pulver.

### 4-(4-Jod-2-methoxyphenyl)-4-methyl-2-oxo-valeriansäure

Zu 24,2 mmol Methylmagnesiumbromid in 23 ml Diethylether werden 3,2 g 4-Jod-2-methoxybenzoesäure-methylester in 10 ml Diethylether gegeben. Nach 20 Stunden wird Ammoniumchloridlösung zugegeben, die Etherphase abgetrennt, getrocknet und eingedampft. 2,4 g des Rückstands werden in 10 ml Dichlormethan gelöst, mit 714 mg 2-Trimethylsilyloxy-acrylsäure-ethylester versetzt, auf -70°C gekühlt und mit 0,27 ml Zinn(IV)chlorid versetzt. Nach 15 Minuten wird die Lösung auf Kaliumcarbonatlösung gegeben. Nach Extraktion mit Diethylether wird die organische Phase mit Wasser gewaschen, getrocknet und eingedampft. 500 mg des so erhaltenen 4-(4-Jod-2-methoxyphenyl)-4-methyl-2-oxo-valeriansäureethylesters werden mit 8,6 ml 1 M Natriumhydroxid in Ethanol / Wasser (2 : 1, v/v) 3 Stunden bei Raumtemperatur gerührt. Nach Zugabe von Wasser wird mit Diethylether extrahiert, die Wasserphase mit 1 m Salzsäure angesäuert und mit Diethylether extrahiert. Nach Trocknen und Eindampfen erhält man 410 mg 4-(4-Jod-2-methoxyphenyl)-4-methyl-2-oxo-valeriansäure als gelbliches Öl.

### 4-(5-Fluor-2-methoxyphenyl)-4-methyl-2-oxo-valeriansäure

wird analog dem vorstehenden Ausführungsbeispiel erhalten, Fp. 58-60°C.

### 4-(4-Brom-2-methoxyphenyl)-2-oxo-valeriansäure

wird analog dem vorstehenden Ausführungsbeispiel als Öl erhalten.

### 4-(2,3-Dihydro-7-benzofuranyl)-4-methyl-2-oxo-valeriansäure

Aus 1,7 g 2,3-Dihydro-benzofuran-7-carbonsäuremethylester in 35 ml Diethylether und 7 ml einer 3 molaren Lösung von Methylmagnesiumchlorid in Tetrahydrofuran werden in üblicher Weise 1,69 g 1-(2,3-Dihydro-7-benzofuranyl)-1-methyl-ethanol erhalten. Dieses Produkt wird analog dem für 4-(4-Jod-2-methoxyphenyl)-4-methyl-2-oxo-valeriansäure beschriebenen Verfahren umgesetzt. Man erhält 1,8 g der Titelverbindung als gelbbraunes Öl.

### 4-(3-Chlor-2-methoxyphenyl)-2-oxo-valeriansäure

Zu 10 g 3-Chlorsalicylsäure und 18 g Kaliumcarbonat in 88 ml Dimethylformamid werden 8,2 ml Methyljodid zugesetzt und das Gemisch über Nacht gerührt. Man verdünnt mit Wasser, extrahiert mit Ethylacetat, trocknet die organische Phase (Na₂SO₄) und dampft ei. Der Rückstand wird am Kugelrohr destilliert. Man erhält 10 g 3-Chlor-2-methoxybenzoesäuremethylester, Kp. 100°C / 0,17 mbar.

Dieser Ester wird analog dem für 4-(4-Jod-2-methoxyphenyl)-4-methyl-2-oxo-valeriansäure beschriebenen Verfahren umgesetzt. Man erhält 8 g der Titelverbindung als hellgelbes Öl.

### 4-(6-Fluor-2-methoxyphenyl)-2-oxo-valeriansäure

Aus 18 g 6-Fluorsalicylsäure werden analog dem für 4-(3-Chlor-2-methoxyphenyl)-2-oxo-valeriansäure beschriebenen Verfahren 18 g der Titelverbindung als hellgelbes Öl erhalten.

### 4-Toluolsulfonsäure-[4-(5-fluor-2-methoxyphenyl)-2-hydroxy-4-methyl-2-trifluormethyl-pentyl)-ester

810 mg 4-(5-Fluor-2-methoxyphenyl)-2-hydroxy-4-methyl-2-trifluormethyl-valeriansäure werden in 20 ml Ether mit 190 mg Lithium-aluminiumhydrid reduziert. Nach Zugabe von wässeriger Natriumhydrogencarbonatlösung wird die Etherphase abgetrennt, getrocknet (Na₂SO₄), eingedampft und der Rückstand am Kugelrohr destilliert. Man erhält 700 mg 2-Hydroxy-4-(5-fluor-2-methoxyphenyl)-4-methyl-2-trifluormethyl-pentanol, die in 7 ml Pyridin gelöst und bei 0°C mit 440 mg 4-Toluolsulfonsäurechlorid versetzt werden. Nach 2 Tagen bei 0°C wird im Vakuum eingeengt, zwischen 1 M Salzsäure und Ethylacetat verteilt, die Ethylacetatphase noch mehrfach mit 1 M Salzsäure gewaschen, getrocknet (Na₂SO₄) und eingedampft. Man erhält so die Titelverbindung, Fp. 93-94°C.

### 4-Toluolsulfonsäure-[4-(5-fluor-2-methoxy-phenyl)-2-hydroxy-2-trifluormethylpentyl]-ester

wird analog der vorhergehenden Vorschrift hergestellt, Fp. 48-50°C.

### 2-[2-(5-Fluor-2-methoxyphenyl)-2-methyl-propyl]-2-trifluormethyl-oxiran

3,5 g 2-Hydroxy-4-(5-fluor-2-methoxyphenyl)-4-methyl-2-trifluormethyl-pentanol und 3,45 g Triphenylphosphin werden in 50 ml Tetrahydrofuran gelöst und bei 0°C mit 2,5 ml Azodicarbonsäure-diethylester in 3 Portionen versetzt. Nach einer Stunde bei 0°C wird noch 16 Stunden bei Raumtemperatur gerührt, dann 7 Stunden am Rückfluß gekocht und erneut 48 Stunden bei Raumtemperatur gerührt. Nach Eindampfen wird ein Hexan-Isopropylether-Gemisch zugegeben, filtriert und das Filtrat an Kieselgel chromatographiert. Das mit Hexan / Ethylacetat eluierte Produkt wird am Kugelrohr destilliert, Kp. 100°C / 0,5 mbar.

### 2-[2-(4-Brom-2-methoxyphenyl)-2-methyl-propyl]-2-trifluormethyl-oxiran

wird analog dem vorhergehenden Beispiel erhalten, Kp. 120°C / 0,04 mbar.

### 4-Brom-5-aminophthalid

23 g 3-Brom-4-nitro-1,2-xylol werden in 200 ml Pyridin und 600 ml Wasser suspendiert und bei 60°C portionsweise mit 260 g Kaliumpermanganat versetzt, wobei die Temperatur auf 90°C steigt. Man erwärmt noch 2 Stunden auf 95°C , filtriert, säuert das Filtrat mit Salzsäure an und extrahiert mit Diethylether. Nach Eindampfen des Lösemittels erhält man 27 g 3-Brom-4-nitrophthalsäure.

12 g der Säure werden 15 Minuten auf 220°C erhitzt und danach am Kugelrohr destilliert. Bei 0,03 hPa destillieren 10 g 3-Brom-4-nitrophthalsäureanhydrid über.

Das Anhydrid wird in 120 ml Dimethylformamid gelöst und bei 0°C mit 78,8 ml einer 0,5 M Lösung von Natriumborhydrid in Dimethylformamid langsam versetzt. Nach drei Stunden bei 0°C wird vorsichtig 2 n Salzsäure zugesetzt und mit Ethylacetat extrahiert. Nach Waschen mit Kaliumbicarbonatlösung, Trocknen (Na₂SO₄) und Eindampfen der Ethylacetatphase erhält man 6,6 g 4-Brom-5-nitrophthalid.

6,6 g 4-Brom-5-nitrophthalid werden in 45 ml Ethanol gelöst und zu einem auf 60°C erwärmten und gut gerührten Gemisch von 65 g Eisen-II-sulfat, 220 ml Wasser und 65 ml Ammoniak (33%) getropft. Nach 2 Stunden bei 60°C wird das Gemisch fünfmal mit 200 ml Diethylether ausgerührt. Die Diethyletherphasen werden eingedampft. Als Rückstand werden 4,1 g 4-Brom-5-aminophthalid erhalten, Fp.176-180°C.

### 6-Brom-5-aminophthalid

4-Brom-5-nitrophthalsäureanhydrid wird analog dem vorstehend beschriebenen Verfahren aus 4-Brom-5-nitro-1,2-xylol hergestellt.

Durch Kochen mit Ethanol wird daraus ein Gemisch von 2-Brom-6-ethoxycarbonyl-3-nitro-benzoesäure und 3-Brom-2-ethoxycarbonyl-4-nitro-benzoesäure gewonnen.

Zu 7,2 ml einer 0,66 m Lösung von Dimethylformamid in Dichlormethan werden bei 0°C vorsichtig 1,2 ml Oxalylchlorid getropft. Die Lösung wird 1 Stunde bei 0°C und 5 Minuten bei Raumtemperatur gerührt. Nach Eindampfen im Vakuum wird der Rückstand in 7 ml Acetonitril suspendiert, auf -35°C gekühlt und tropfenweise mit 1,5 g des Estergemisches versetzt. Nach einer Stunde bei derselben Temperatur wird auf -70°C abgekühlt und 2,4 ml einer 2 m Lösung von Natriumborhydrid in Dimethylformamid zugetropft. Man rührt 20 Stunden bei Raumtemperatur, setzt Wasser zu, alkalisiert mit Kaliumcarbonat und extrahiert mit Diethylether. Die Diethylether-Phase wird getrocknet (Na₂SO₄) und eingedampft. Man erhält ein Gemisch von 5-Brom-6-nitrophthalid und 6-Brom-5-nitrophthalid, das an Kieselgel mit Hexan / Ethylacetat ( 95:5) getrennt wird.

Die Reduktion zum Aminophthalid erfolgt wie oben beschrieben. Man erhält 6-Brom-5-aminophthalid, Fp. 235-241°C.

Analog erhält man die Phthalide der Tabelle 2.

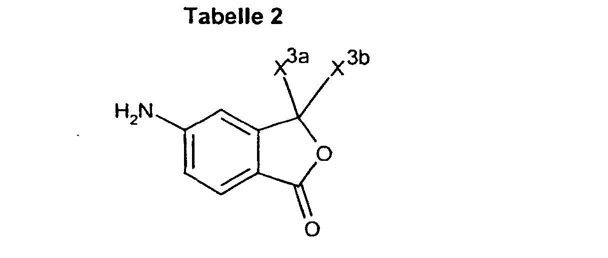

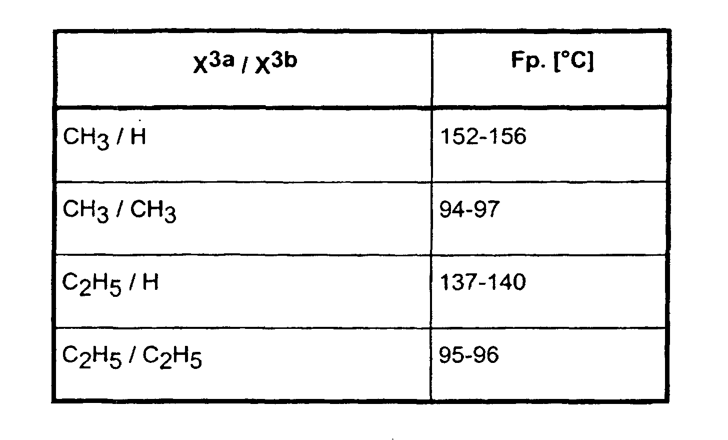

### 5-Acetamido-phthalid

3 g 5-Amino-phthalid, 10 ml Acetanhydrid und 30 ml Tetrahydrofuran werden 1 Stunde im Rückfluß gekocht. Die nach Erkalten ausgefallenen Kristalle werden abgesaugt und mit Isopropylether gewaschen. Man erhält 3,3 g der Titelverbindung, Fp. >300°C.

### 6-Amino-4-methyl-2,3-benzoxazin-1-on

60 g 2-Methyl-5-nitroacetophenon, 38,5 g 2,2-Dimethyl-1,3-propandiol und 6 g p-Toluolsulfonsäure werden in 1 l Toluol mit einem Wasserabscheider bis zum Ende der Wasserentwicklung gekocht. Die Lösung wird mit Kalumbicarbonat gewaschen, getrocknet (Na₂SO₄) und eingedampft. Aus Pentan erhält man 71,7 g des kristallinen Ketals.

Dieses wird in 1,5 l Pyridin und 4,5 l Wasser mit 350 g Kaliumpermanganat, wie oben bei der Herstellung von 4-Brom-5-aminophthalid beschrieben, oxidiert. Man erhält 56,4 g 4-Nitro-2-(2,5,5-trimethyl-1,3-dioxan-2-yl)-benzoesäure.

52 g der Säure werden in 500 ml Methanol und 500 ml Ethylacetat mit 10 g Palladium/Kohle (10%) hydriert. Man erhält aus Pentan 45,5 g der kristallinen Aminoverbindung.

10 g des Amins werden mit 100 ml konzentrierter Salzsäure 2 Stunden am Rückfluß gekocht. Das Lösemittel wird im Vakuum eingedampft und der Rückstand mit 15,7 g Hydroxylamin Hydrochlorid, 8,4 g Kaliumhydroxid, 120 ml Ethanol und 50 ml Wasser 12 Stunden am Rückfluß gekocht. Man verdünnt mit Wasser und saugt die Kristalle ab. Nach Trocknen erhält man 3,5 g 6-Amino-4-methyl-2,3-benzoxazin-1-on, Fp. 291-296°C.

### 6-Amino-4-ethyl-2,3-benzoxazin-1-on

wird analog aus 2-Methyl-5-nitropropiophenon erhalten, Fp 89-93°C.

### 6-Acetamido-4-methyl-2,3-benzoxazin-1-on

Diese Verbindung wird analog dem 5-Acetamido-phthalid aus 6-Amino-4-methyl-2,3-benzoxazin-1-on erhalten, nur wird 6 Tage am Rückfluß gekocht und dann Wasser zugegeben und mit Ethylacetat extrahiert. Nach Trocknen und Eindampfen des Lösemittels erhält man die Titelverbindung als Kristalle, Fp.223-229°C.

### 5-[4-(4-Jod-2-methoxyphenyl)-4-methyl-2-oxo-valeroylamino)-phthalid

1,7 g 4-(4-Jod-2-methoxyphenyl)-4-methyl-2-oxo-valeriansäure werden in 25 ml Dimethylacetamid gelöst und unter Argon bei -8°C mit 0,37 ml Thionylchlorid versetzt. Nach 20 Minuten Rühren bei -3 bis +3°C werden 700 mg 5-Aminophthalid zugegeben. Man rührt 1,5 Stunden bei Raumtemperatur, versetzt dann mit Wasser, extrahiert mit Ethylacetat, wäscht die organische Phase mit Wasser, trocknet (Na₂SO₄) und erhält nach Eindampfen des Lösemittels und Chromatograhie des Rohproduktes an Kieselgel mit Hexan / Ethylacetat (80 : 20) 1,5 g 5-[4-(4-Jod-2-methoxyphenyl)-4-methyl-2-oxo-valeroylamino)-phthalid als Schaum.

### 5-[4-(4-Brom-2-methoxyphenyl)-4-methyl-2-oxo-valeroylamino)-phthalid

wurde analog dem für 5-[4-(4-Jod-2-methoxyphenyl)-4-methyl-2-oxo-valeroylamino)-phthalid beschriebenen Verfahren aus 5-Aminophthalid und 4-(4-Brom-2-methoxyphenyl)-4-methyl-2-oxo-valeriansäure erhalten, Fp. 136-140°C.

### 5-[4-(3-Chlor-2-methoxyphenyl)-4-methyl-2-oxo-valeroylamino)-phthalid

wurde analog dem für 5-[4-(4-Jod-2-methoxyphenyl)-4-methyl-2-oxo-valeroylamino)-phthalid beschriebenen Verfahren aus 5-Aminophthalid und 4-(3-Chlor-2-methoxyphenyl)-4-methyl-2-oxo-valeriansäure als beigefarbener Schaum erhalten.

### 5-[4-(6-Fluor-2-methoxyphenyl)-4-methyl-2-oxo-valeroylamino)-phthalid

wurde analog dem für 5-[4-(4-Jod-2-methoxyphenyl)-4-methyl-2-oxo-valeroylamino)-phthalid beschriebenen Verfahren aus 5-Aminophthalid und 4-(6-Fluor-2-methoxyphenyl)-4-methyl-2-oxo-valeriansäure erhalten, Fp. 175-179°C.

### 5-{3-[1-(2-Methoxyphenyl)-cyclopropyl]-2-oxo-propionylamino}-phthalid

wurde analog dem für 5-[4-(4-Jod-2-methoxyphenyl)-4-methyl-2-oxo-valeroylamino)-phthalid beschriebenen Verfahren aus 5-Aminophthalid und 3-[1-(2-Methoxyphenyl)-cyclopropyl]-2-oxo-propionsäure erhalten, Fp. 190-202°C.

### 5-{3-[1-(5-Fluor-2-methoxyphenyl)-cyclopropyl]-2-oxo-propionylamino}-phthalid

wurde analog dem für 5-[4-(4-Jod-2-methoxyphenyl)-4-methyl-2-oxo-valeroylamino)-phthalid beschriebenen Verfahren aus 5-Aminophthalid und 3-[1-(5-Fluor-2-methoxyphenyl)-cyclopropyl]-2-oxo-propionsäure erhalten, Fp. 190-193°C.

### 5-[4-(2,3-Dihydro-7-benzofuranyl)-4-methyl-2-oxo-valeroylamino]-phthalid

wurde analog dem für 5-[4-(4-Jod-2-methoxyphenyl)-4-methyl-2-oxo-valeroylamino)-phthalid beschriebenen Verfahren aus 5-Aminophthalid und 4-(2,3-Dihydro-7-benzofuranyl)-4-methyl-2-oxo-valeriansäure als weißer Schaum erhalten.

### 6-[4-(5-Fluor-2-methoxyphenyl)-4-methyl-2-oxo-valeroylamino]-4-methyl-2,3-benzoxazin-1-on

wurde analog dem für 5-[4-(4-Jod-2-methoxyphenyl)-4-methyl-2-oxo-valeroylamino)-phthalid beschriebenen Verfahren aus 6-Amino-4-methyl-2,3-benzoxazin-1-on und 4-(5-Fluor-2-methoxyphenyl)-4-methyl-2-oxo-valeriansäure erhalten, Fp. 171-173°C.

### 4-Ethyl-6-[4-(5-fluor-2-methoxyphenyl)-4-methyl-2-oxo-valeroylamino)-2,3-benzoxazin-1-on

wurde analog dem für 5-[4-(4-Jod-2-methoxyphenyl)-4-methyl-2-oxo-valeroylamino)-phthalid beschriebenen Verfahren aus 6-Amino-4-ethyl-2,3-benzoxazin-1-on und 4-(5-Fluor-2-methoxyphenyl)-4-methyl-2-oxo-valeriansäure erhalten, Fp. 157-158°C.

### 6-[4-(6-Fluor-2-methoxyphenyl)-4-methyl-2-oxo-valeroylamino]-4-methyl-2,3-benzoxazin-1-on

wurde analog dem für 5-[4-(4-Jod-2-methoxyphenyl)-4-methyl-2-oxo-valeroylamino)-phthalid beschriebenen Verfahren aus 6-Amino-4-methyl-2,3-benzoxazin-1-on und 4-(6-Fluor-2-methoxyphenyl)-4-methyl-2-oxo-valeriansäure erhalten, Fp. 178-181°C.

### 6-[1-(5-Fluor-2-methoxyphenyl)-cyclopropyl]-2-oxo-propionylamino}-4-methyl-2,3-benzoxazin-1-on

wurde analog dem für 5-[4-(4-Jod-2-methoxyphenyl)-4-methyl-2-oxo-valeroylamino)-phthalid beschriebenen Verfahren aus 6-Amino-4-methyl-2,3-benzoxazin-1-on und 3-[1-(5-Fluor-2-methoxyphenyl)-cyclopropyl]-2-oxo-propionsäure erhalten, Fp. 222-227°C.

### 6-[4-(2,3-Dihydro-7-benzofuranyl)-4-methyl-2-oxo-valeroylamino]-4-methyl-2,3-benzoxazin-1-on

wurde analog dem für 5-[4-(4-Jod-2-methoxyphenyl)-4-methyl-2-oxo-valeroylamino)-phthalid beschriebenen Verfahren aus 6-Amino-4-methyl-2,3-benzoxazin-1-on und 4-(2,3-Dihydro-7-benzofuranyl)-4-methyl-2-oxo-valeriansäure erhalten, Fp. 171-177°C.

Die nachfolgenden Beispiele dienen der näheren Erläuterung der Erfindung. Weitere Verbindungen lassen sich durch Verwendung homologer / analoger Reagenzien herstellen. Die erforderlichen Ausgangsverbindungen sind vorstehend unter den "Ausgangsverbindungen" beschrieben.

### Beispiel 1 (Verfahren1)

### 5-{2-Hydroxy-3-[1-(2-methoxyphenyl)-cyclopropyl]-2-trifluormethyl-propionylamino}-phthalid

500 mg 5-{3-[1-(2-Methoxyphenyl)-cyclopropyl]-2-oxo-propionylamino}-phthalid werden unter Argon in 15 ml Dimethylformamid gelöst und unter Eiskühlung mit 0,77 ml Trifluormethyl-trimethylsilan und 500 mg Caesiumcarbonat versetzt. Nach 18 Stunden Rühren bei Raumtemperatur werden 5 ml einer 1 M Lösung von Tetrabutylammoniumfluorid in Tetrahydrofuran und einige Tropfen Wasser zugesetzt und eine Stunde bei Raumtemperatur gerührt. Nach Zusatz von 100 ml Wasser wird mit Ethylacetat extrahiert, die organische Phase getrocknet (Na₂SO₄) und eingedampft.
Das Rohprodukt wird an Kieselgel chromatographiert. Mit Hexan / Ethylacetat (80 : 20) wird die Titelverbindung rein erhalten.
Das Enantiomerengemisch wird durch Chromatographie an chiralem Trägermaterial (CHIRALPAK AD®, Fa DAICEL) mit Hexan / 2-Propanol / Ethanol (900 : 25 :2 5, vw) getrennt. Man erhält so das
(+)-Enantiomer mit Fp. 200-208°C, [α]_{D} +106,9° (c = 0,5, CHCl₃),
(-)-Enantiomer mit Fp. 195-208°C, [α]_{D} -104,9°(c = 0,5, CHCl₃).

### Beispiel 2

### 5-{3-[1-(5-Fluor-2-methoxyphenyl)-cyclopropyl]-2-hydroxy-2-trifluormethyl-propionylamino}-phthalid

wird analog Beispiel 1 aus 5-{3-[1-(5-Fluor-2-methoxyphenyl)-cyclopropyl]-2-oxo-propionylamino}-phthalid erhalten. Fp. 170-179°C (Racemat).

### Beispiel 3 (Verfahren 2)

### 5-[2-Hydroxy-4-(2-methoxyphenyl)-4-methyl-2-trifluormethyl-valeroylamino]-phthalid

400 mg 2-Hydroxy-4-(2-methoxyphenyl)-4-methyl-2-trifluormethyl-valeriansäure werden in 5 ml Dimethylacetamid bei 0°C mit 0,23 ml Thionylchlorid versetzt. Nach 30 Minuten Rühren bei 0°C werden 390 mg 5-Aminophthalid in 2 ml Dimethylacetamid zugegeben und das Gemisch wird weitere 4 Stunden bei dieser Temperatur gerührt. Dann wird Eiswasser zugesetzt, mit Ethylacetat extrahiert und nach Trocknen (Na₂SO₄) wird das Rohprodukt an Kieselgel chromatographiert. Mit Hexan / Ethylacetat (50 : 50) wird die Titelverbindung kristallin erhalten, Fp. 134-135°C.
Nach Racemattrennung erhält man das
(+)-Enantiomer mit Fp. 135-136°C, [α]_{D} +192,2° °(c=1, CHCl₃),
(-)-Enantiomer mit Fp. 136-137°C, [α]_{D} -194,8° °(c=1, CHCl₃).

Analog werden die Verbindung der Tabelle 3 erhalten.

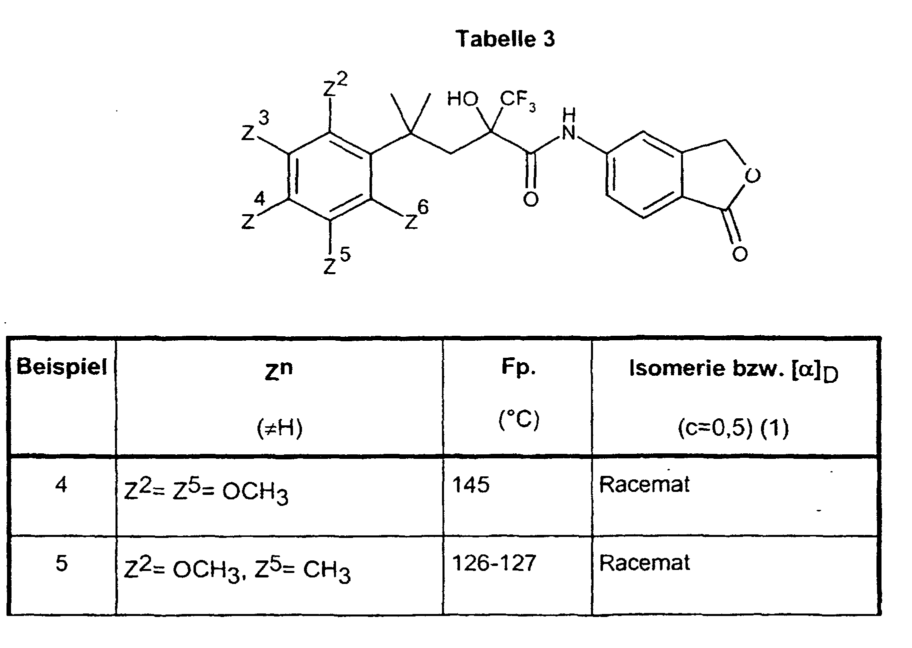

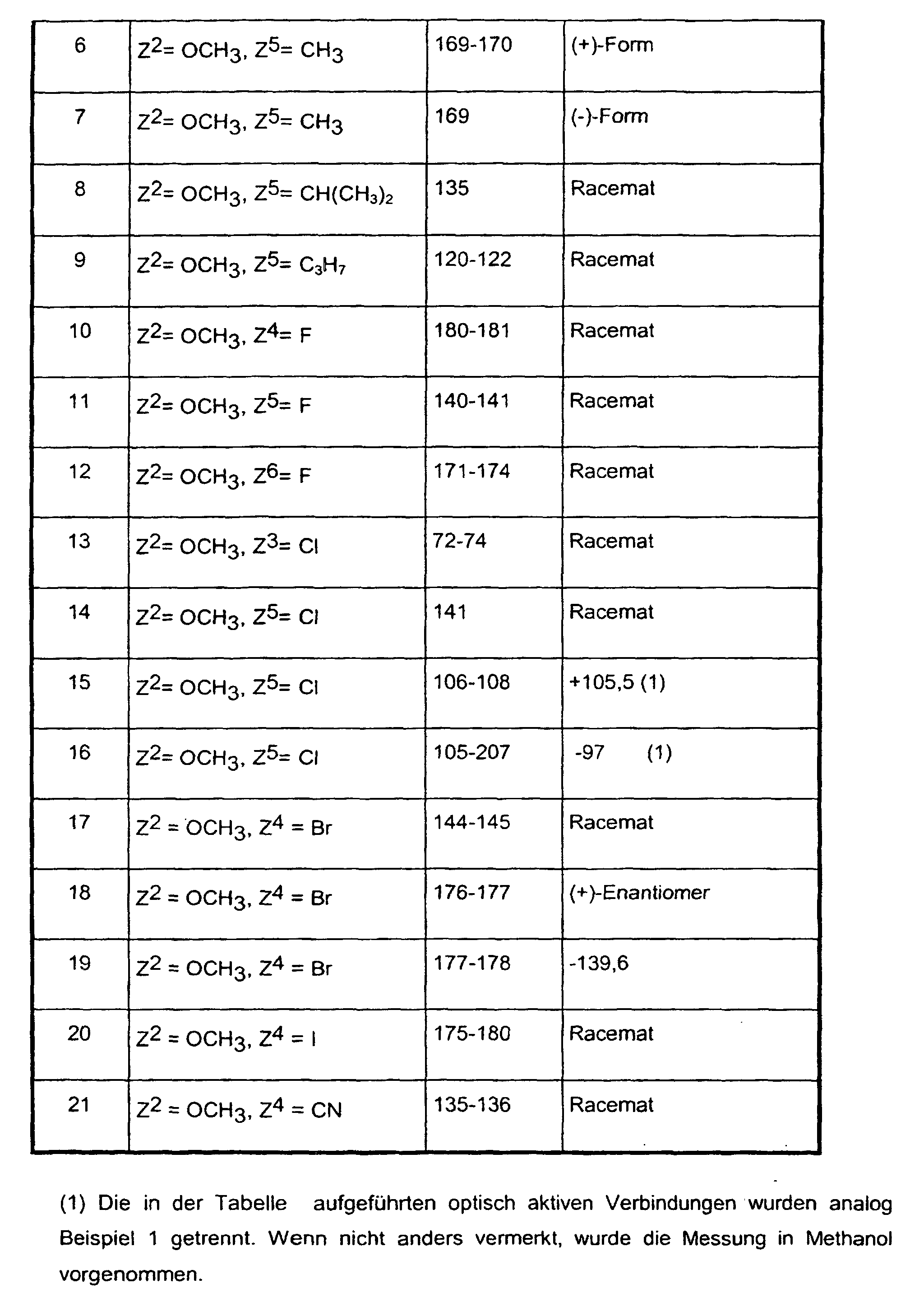

Verwendet man in Beispiel 3 anstelle des 5-Aminophthalids 6-Amino-4-methyl-2,3-benzoxazin-1-on bzw. 6-Amino-4-ethyl-2,3-benzoxazin-1-on so erhält man die in Tabelle 4 zusammengestellten Beispiele.

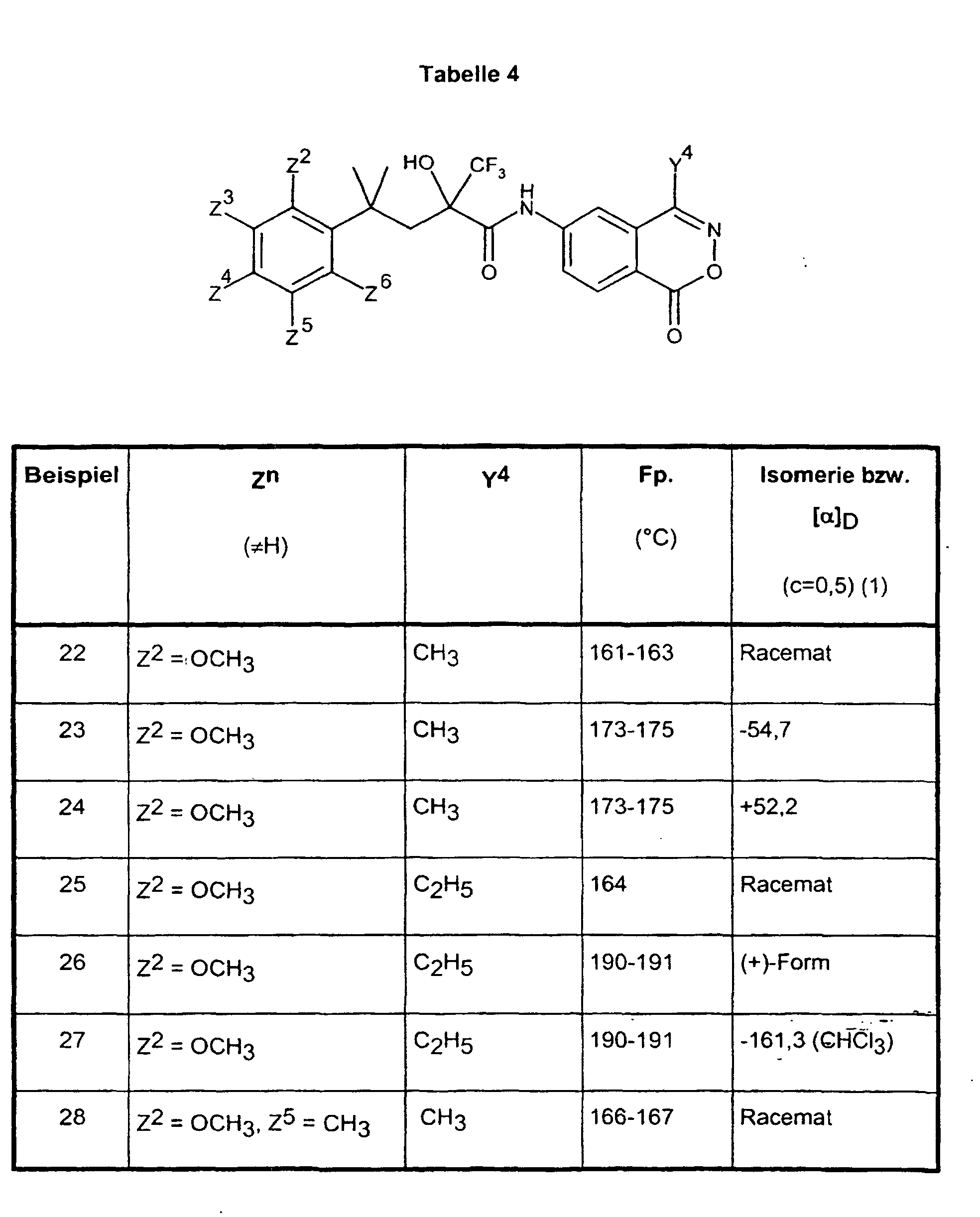

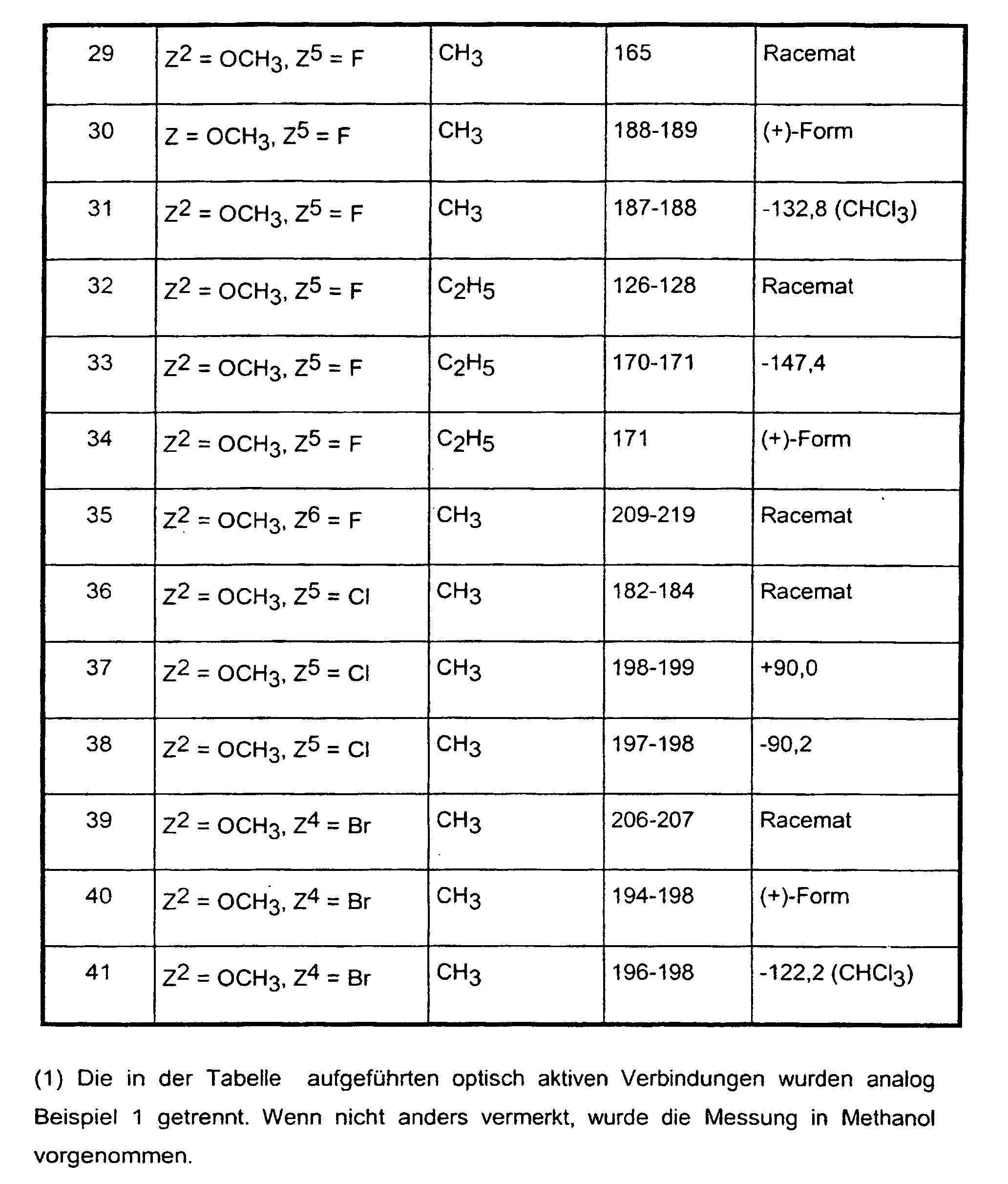

### Beispiel 42

### 5-[2-Hydroxy-4-(5-fluor-2-methoxyphenyl)-4-methyl-2-trifluormethyl-pentylamino]-phthalid

688 mg 5-Acamidophthalid in 15 ml Dimethylformamid werden unter Argon bei 0°C mit 108 mg einer 80 prozentigen Natriumhydrid / Ölsuspension versetzt. Nach 10 Minuten Rühren bei dieser Temperatur werden 556 mg 4-Toluolsulfonsäure-[2-hydroxy-4-(5-fluor-2-methoxyphenyl)-2-trifluormethy-pentyl]-ester zugesetzt. Nach 16 Stunden Rühren bei 60°C wird auf 1 M Salzsäure gegeben, mit Kaliumcarbonat neutralisiert und mit Ethylacetat extrahiert. Nach Trocknen (Na₂SO₄) wird das Rohprodukt an Kieselgel chromatographiert. Mit Hexan / Ethylacetat (60 : 40) wird die Titelverbindung kristallin erhalten, Fp. 148-149°C.

### Beispiel 43

### 6-[2-Hydroxy-4-(5-fluor-2-methoxyphenyl)-4-methyl-2-trifluormethyl-pentylamino]-4-methyl-2,3-benzoxazin-1-on

584 mg 2-[2-(5-Fluor-2-methoxyphenyl)-2-methyl-propyl]-2-trifluormethyl-oxiran, 282 mg 6-Amino-4-methyl-2,3-benzoxazin-1-on und 1 ml 1,3-Dimethyl-3,4,5,6-tetrahydro-2[1H]-pyrimidinon werden 6 Stunden auf 120°C erwärmt. Nach Zugabe von 1 ml Tetrahydrofuran chromatographiert man an Kieselgel und eluiert die Titelverbindung mit Hexan / Ethylacetat / Tetrahydrofuran (55 : 40 : 5), Fp. 178-179°C

### Beispiel 44

### 5-{3-[1-(5-Fluor-2-hydroxyphenyl)-cyclopropyl]-2-hydroxy-2-trifluormethyl-propionylamino}-phthalid

225 mg 5-{3-[1-(5-Fluor-2-methoxyphenyl)-cyclopropyl]-2-hydroxy-2-trifluormethyl-propionylamino}-phthalid werden in 4,5 ml Dichlormethan bei 0°C mit 2,48 ml einer 1 molaren Lösung von Bortribromid in Dichlormethan versetzt. Nach 3 Stunden Rühren bei 0°C wird das Gemisch auf Wasser gegeben, mit Ethylacetat extrahiert, die organische Phase getrocknet (Na₂SO₄) und eingedampft. Nach Verreiben des Rückstands mit Hexan erhält man die Titelverbindung in kristalliner Form, Fp. 196-199°C.

### Beispiel 45

### 6-{3-[1-(5-Fluor-2-hydroxyphenyl)-cyclopropyl]-2-hydroxy-2-trifluormethyl-propionylamino}-4-methyl-2,3-benzoxazin-1-on

wird analog 5-{3-[1-(5-Fluor-2-hydroxyphenyl)-cyclopropyl]-2-hydroxy-2-trifluormethyl-propionylamino}-phthalid aus 6-{3-[1-(5-Fluor-2-methoxyphenyl)-cyclopropyl]-2-hydroxy-2-trifluormethyl-propionylamino}-4-methyl-2,3-benzoxazin-1-on erhalten. Fp. 236-244°C.

### Beispiel 46

### 6-[2-Hydroxy-4-(5-fluor-2-hydroxyphenyl)-4-methyl-2-trifluormethyl-valeroylamino]-4-methyl-2,3-benzoxazin-1-on

wird analog 5-{3-[1-(5-Fluor-2-hydroxyphenyl)-cyclopropyl]-2-hydroxy-2-trifluormethyl-propionylamino}-phthalid aus 6-[2-Hydroxy-4-(5-fluor-2-methoxyphenyl)-4-methyl-2-trifluormethyl- valeroylamino]-4-methyl-2,3-benzoxazin-1-on erhalten. Fp. 234-236°C,
(+)-Enantiomer, Fp. 230-234°C [α]_{D} +34° °(c=0,5)
(-)-Enantiomer, Fp. 230-232°C, [α]_{D} -34.1° °(c=0,5).

### Beispiel 47

### 6-[2-Hydroxy-4-(2-hydroxyphenyl)-4-methyl-2-trifluormethyl-valeroylamino]-4-ethyl-2,3-benzoxazin-1-on

wird analog 5-{3-[1-(5-Fluor-2-hydroxyphenyl)-cyclopropyl]-2-hydroxy-2-trifluormethyl-propionylamino}-phthalid aus 6-[2-Hydroxy-4-(2-methroxyphenyl)-4-methyl-2-trifluormethyl- valeroylamino]-4-ethyl-2,3-benzoxazin-1-on erhalten. Fp. 164°C,
(+)-Enantiomer mit Fp. 191-192°C, [α]_{D} +161,5° °(c=0,5, CHCl₃)
(-)-Enantiomer mit Fp. 190-191°C, [α]_{D} -161,3° °(c=0,5, CHCl₃)

Analog werden die Beispiele der Tabelle 5A und 5B erhalten.

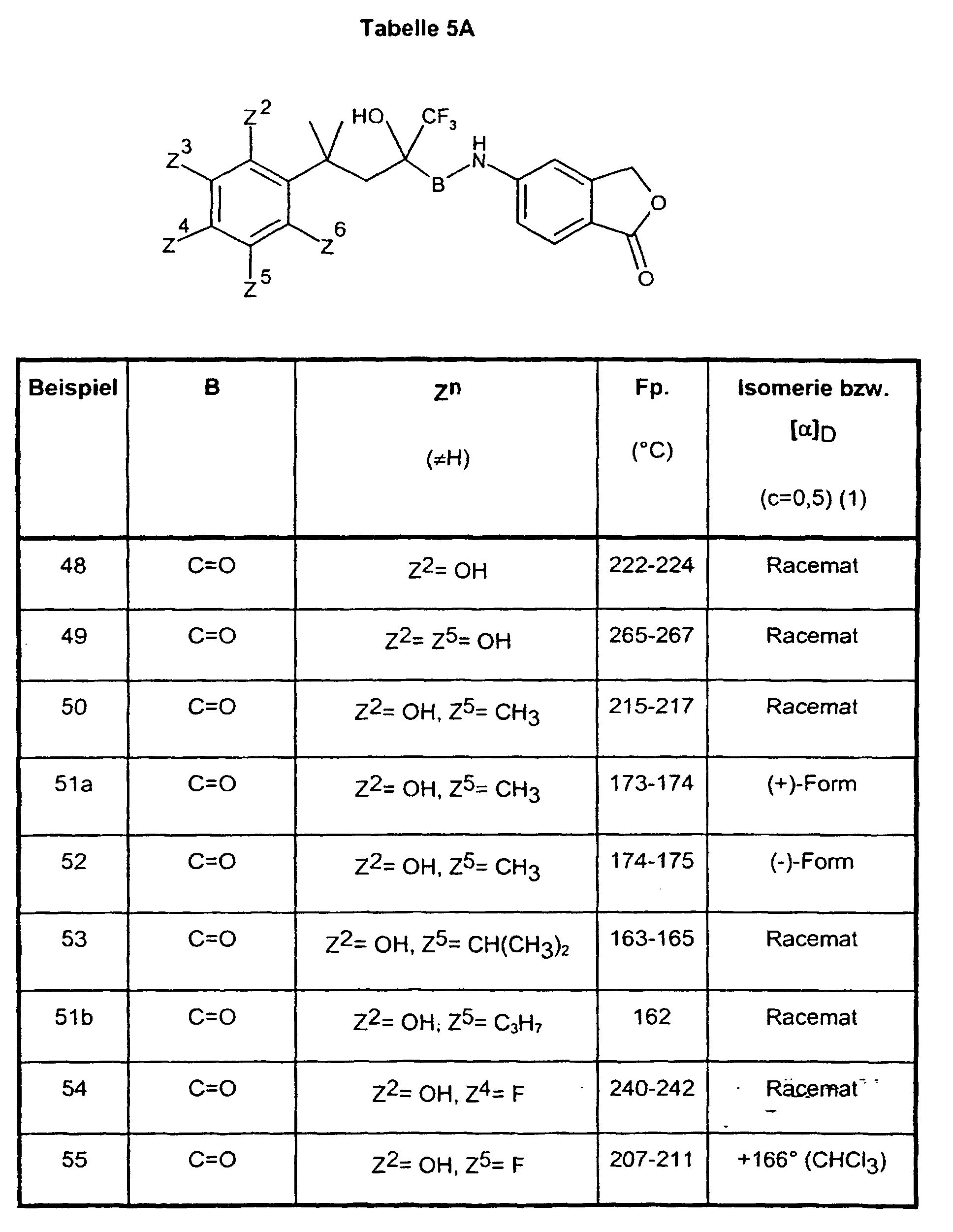

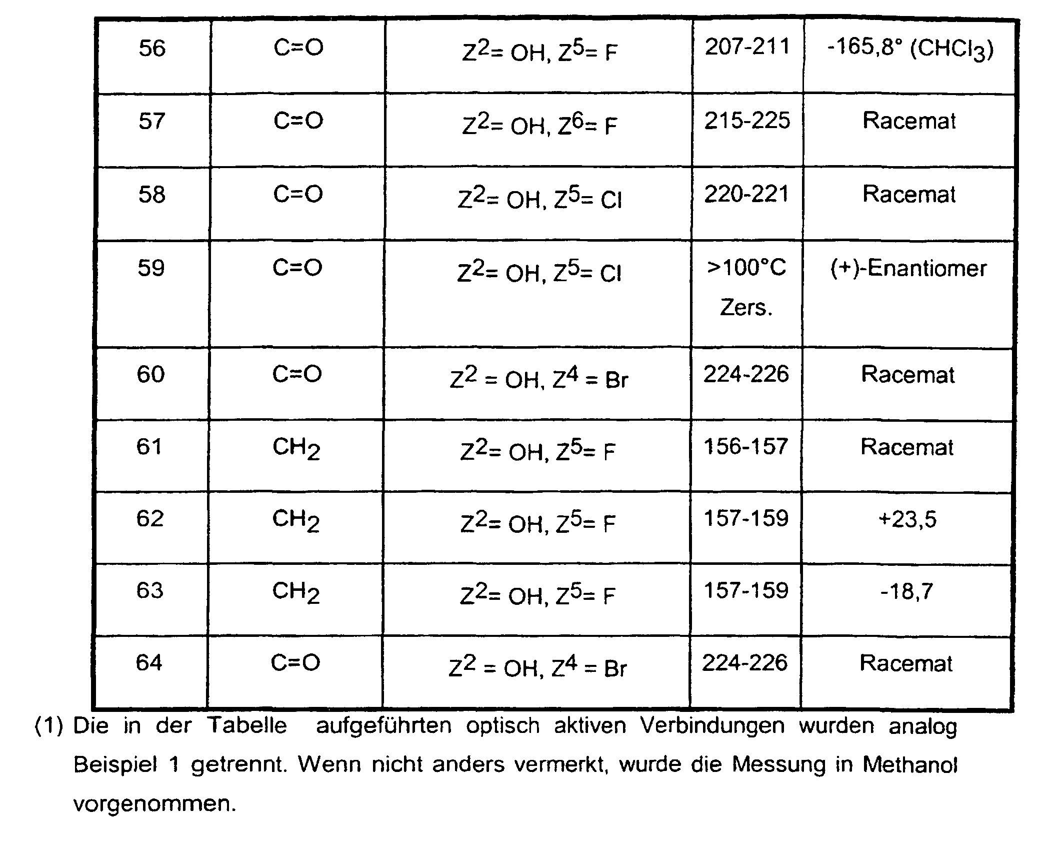

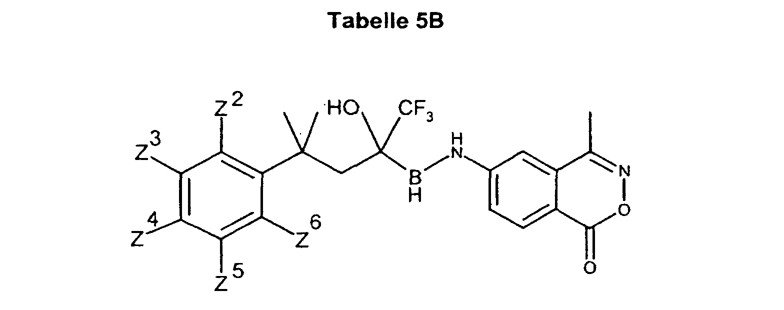

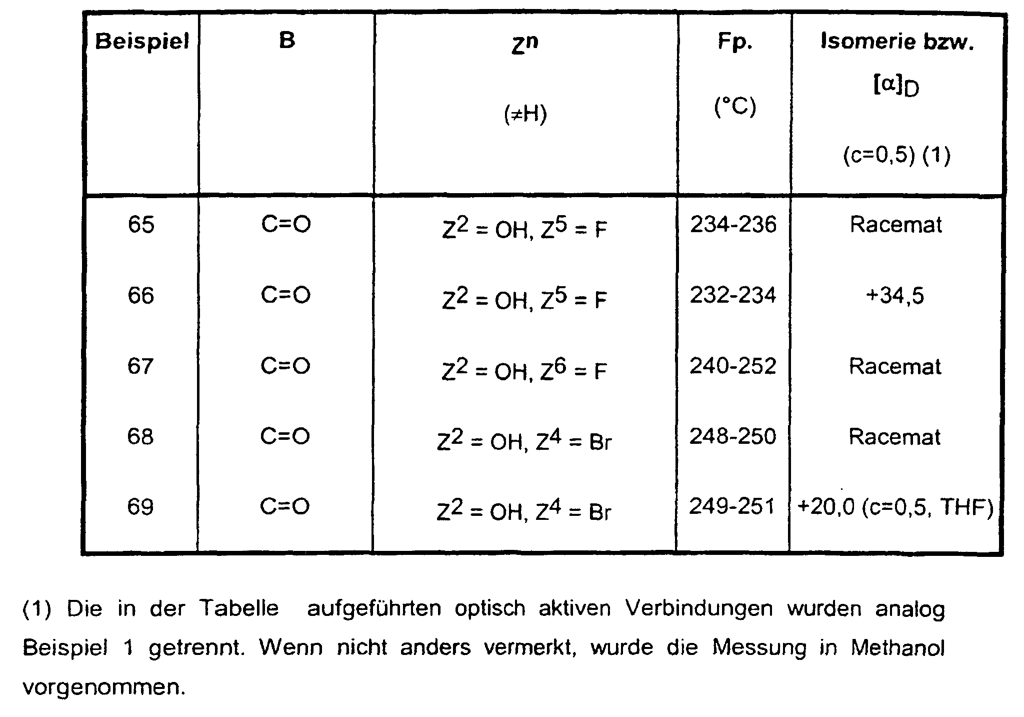

### Beispiel 70 (Verfahren 5)

### 5-[4-(2-Ethoxy-5-fluorphenyl)-2-hydroxy-4-methyl-2-trifluormethyl-valeroylamino]-phthalid

44 mg 5-[4-(5-Fluor-2-hydroxyphenyl)-2-hydroxy-4-methyl-4-2-trifluormethyl-valeroyl-amino]-phthalid werden in 1 ml Dimethylformamid mit 28 mg Kaliumcarbonat und 50 mg Ethyljodid 24 Stunden bei Raumtemperatur gerührt. Man versetzt dann mit Wasser, extrahiert mit Ethylacetat, wäscht die organische Phase mit Wasser, trocknet (Na₂SO₄) und erhält nach Eindampfen des Lösemittels 35 mg 5-[4-(2-Ethoxy-5-fluorphenyl)-2-hydroxy-4-methyl-2-trifluormethyl-valeroylamino]-phthalid, Fp. 108°C.

Analog dem vorstehenden Beispiel wurden die Verbindungen der Tabelle 6 hergestellt.

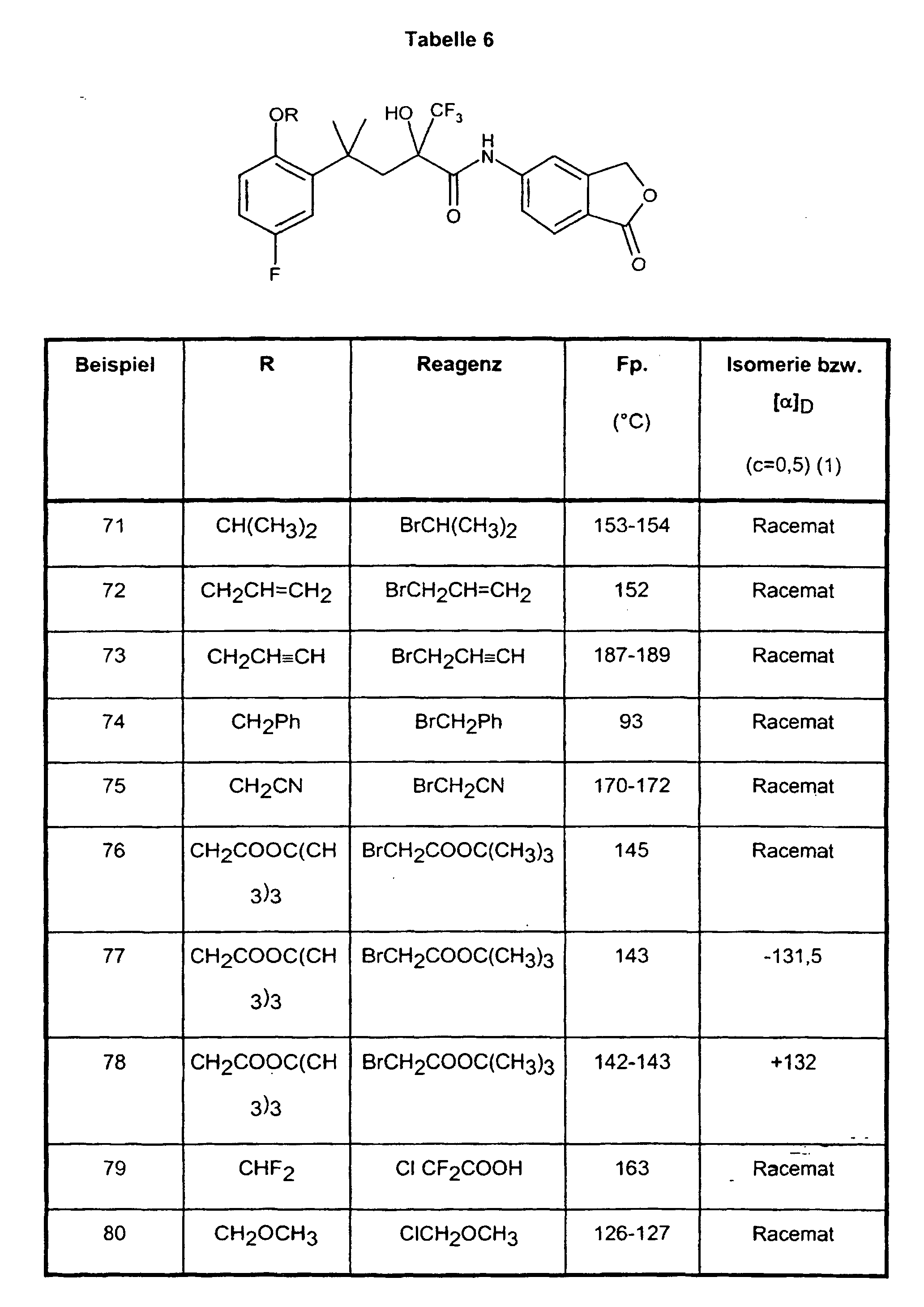

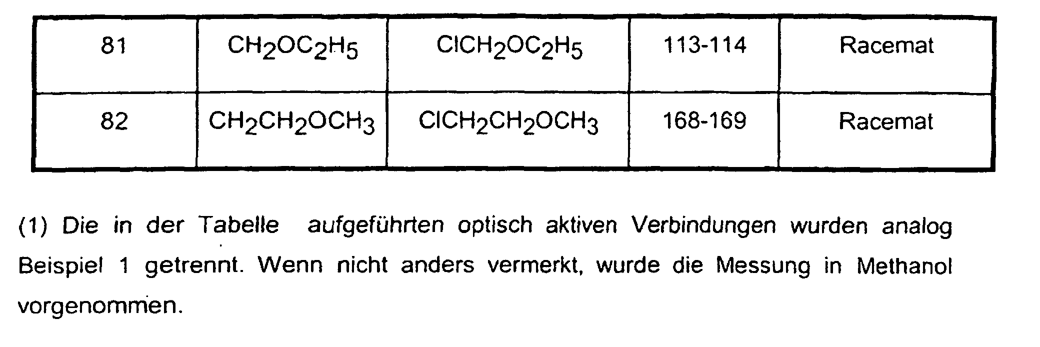

### Beispiel 83

### (-)-4-Brom-5-[4-(5-fluor-2-hydroxyphenyl)-2-hydroxy-4-methyl-2-trifluormethyl-pentylamino]-phthalid

55 mg (-)-5-[4-(5-Fluor-2-hydroxyphenyl)-2-hydroxy-4-methyl-2-trifluormethyl-pentylamino]-phthalid werden unter Stickstoff in 1 ml Dimethylformamid bei 2°C mit 18 mg N-Bromsuccinimid versetzt. Nach 2 Stunden bei dieser Temperatur wird mit 20 ml Ethylacetat verdünnt, mit Wasser extrahiert und die organische Phase getrocknet (Na₂SO₄) und eingedampft. Bei Chromatographie an Kieselgel eluiert man mit Hexan / Ethylacetat (4 : 1) die Titelverbindung in kristalliner Form, Fp. 228-232°C.

### Beispiel 84

### (-)-4-Brom-5-[4-(3-brom-5-fluor-2-hydroxyphenyl)-2-hydroxy-4-methyl-2-trifluormethyl-pentylamino]-phthalid

Verwendet man im vorangehenden Beispiel auf 100 mg (-)-5-[4-(5-Fluor-2-hydroxyphenyl)-2-hydroxy-4-methyl-2-trifluormethyl-pentylamino]-phthalid 44 mg N-Bromsuccinimid so erhält man nach Chromatographie an Kieselgel mit Hexan / Ethylacetat (7 : 3) die Titelverbindung in kristalliner Form, Fp. 144-146°C.

### Beispiel 85

### 5-[4-(2,3-Dihydro-7-benzofuranyl-2-hydroxy-4-methyl-2-trifluormethyl-pentanoyl-amino]-phthalid

wird analog Beispiel 1 aus 5-[4-(2,3-Dihydro-7-benzofuranyl-4-methyl-2-oxo-pentanoyl-amino]-phthalid erhalten. Fp. 182-185°C.

### Beispiel 86

### 6-[4-(2,3-Dihydro-7-benzofuranyl-2-hydroxy-4-methyl-2-trifluormethyl-pentanoyl-amino]-4-methyl-2,3-benzoxazin-1-on

wird analog Beispiel 1 aus 6-[4-(2,3-Dihydro-7-benzofuranyl-4-methyl-2-oxo-pentanoyl-amino]-4-methyl-2,3-benzoxazin-1-on erhalten. Fp. 215-220°C.

### Pharmakologische Beispiele

Im Glucocorticoidrezeptor - (GR) - Bindungstest unter Verwendung von Cytosolpräparationen aus Thymushomogenaten der Ratte und von 10 nM [³H]-Dexamethason als Bezugssubstanz (vgl. Lefebvre et al. J. Steroid. Biochem., 33, 557-563, 1989) zeigen die Verbindungen der Formel 1 eine hohe bis sehr hohe Affinität zum GR (siehe Tabelle).

**Tabelle**

| **für GR-Werte** | |
|---|---|
| **Verbindung** | **IC50 mol/l** |
| l | 2.8·e⁻⁹ |
| li | 2.3·e⁻⁹ |
| lii | 4.6·e⁻⁹ |
| lv | 4.9·e⁻⁹ |
| V | 2.6·e⁻⁹ |
| Dexamethason | 2.0·e⁻⁸ |
| "e" entspricht der Basis der natürlichen Logarithmen. | |

Die in der Tabelle genannten Verbindungen sind die folgenden besonders bevorzugten Verbindungen:
i: 5-[4-(5-Fluor-2-hydroxyphenyl)-2-hydroxy-4-methyl-2-trifluormethyl-valeroylamino]-phthalid
ii: 5-[4-(5-Fluor-2-hydroxyphenyl)-2-hydroxy-4-methyl-2-trifluormethyl-pentylamino]-phthalid
iii: 6-[4-(5-Fluor-2-methoxyphenyl)-2-hydroxy-4-methyl-2-trifluormethyl-valeroylamino]-4-methyl-2,3-benzoxazin-1-on
iv: 6-[4-(4-Brom-2-methoxyphenyl)-2-hydroxy-4-methyl-2-trifluormethyl-valeroylamino]-4-methyl-2,3-benzoxazin-1-on
v: 6-[4-(5-Fluor-2-hydroxyphenyl)-2-hydroxy-4-methyl-2-trifluormethyl-valeroylamino]-4-methyl-2,3-benzoxazin-1-on

Die erfindungsgemäßen Verbindungen der allgemeinen Formel I hemmen die durch Lipopolysaccharid (LPS) ausgelöste Sekretion des Cytokins IL-8 in der menschlichen Monozytenzelline THP-1. Die Konzentration der Cytokine wurden im Überstand mittels kommerziell erhältlicher ELISA-Kits bestimmt (vgl. Welker et al. Int. Arch. Allergy Immunol., 109, 110-115, 1996). Die Verbindungen zeigen in einer Konzentration von 1 µM eine maximale Hemmung der Cytokinsekretion um 50 - 80%.

Die antiinflammatorische Wirkung der Verbindungen der allgemeinen Formel I wurden im Tierexperiment durch Testung in der Crotonöl-induzierten Entzündung in der Ratte und der Maus getestet (vgl. Tubaro et al., Agents Actions, 17, 347-349, 1985). Hierzu wurde den Tieren Crotonöl in ethanolischer Lösung topisch auf die Ohren appliziert. Die Testsubstanzen wurden gleichzeitig oder zwei Stunden vor dem Crotonöl ebenfalls topisch oder systemisch appliziert. Nach 16-24 Stunden wurden das Ohrgewicht als Maß für das entzündliche Ödem, die Peroxidaseaktivität als Maß für die Einwanderungen von Granulozyten und die Elastaseaktivität als Maß für die Einwanderung von neutrophilen Granulozyten gemessen. Die Verbindungen der allgemeinen Formel I hemmen in diesem Test sowohl nach topischer, als auch nach systemischer Applikation die drei oben genannten Entzündungsparameter.
Zur Messung der TAT-Induktion werden die Tiere 6 Stunden nach Gabe der Testsubstanzen getötet, die Leber entnommen und die TAT-Aktivität im Homogenat gemessen (vgl. Diamandstone et al. Anal. Biochemistry, 16, 395-401, 1966). Die Verbindungen hemmten in einer Dosis von 10 - 30 mg/kg Körpergewicht die Ohrentzündung um ca. 50 - 80% und induzierten in diesem Dosisbereich die Tyrosinaminotransferase in der Leber der Tiere um das 1 - 4 fache des Ausgangwertes. Da die Substanzen der allgemeinen Formel auch hohe Affinität am Progesteronrezeptor aufweisen, wurden die neuen Verbindungen auf ihre gestagene Wirkung im Tierexperiment getestet. Hierzu wurde der Schangerschaftserhaltungstest an ovariektomierten Ratten durchgeführt (vgl. Neumann et al. Ameim.-Forsch. (Drug Res.), 34, 296-318, 1984). Hierzu werden weibliche Ratten gedeckt, am Tag acht der Gravidität 2 Stunden nach Substanzapplikation unter Narkose ovariektomiert. An den Tagen 8. - 14 der Schwangerschaft werden die Tiere täglich mit den Testsubstanzen behandelt und am Tag 15 werden die Tiere getötet und pro Tier die Zahl der lebenden und toten Föten ermittelt. Bei leeren Uteri wird die Zahl der Implantationsstellen durch Anfärben mit 10% Ammoniumsulfid-Lösung ermittelt. Die neuen Verbindungen der Formel 1 führten bis zu einer Dosis von 500 µg pro kg Körpergewicht nicht oder nur in geringem Ausmaß zur Schwangerschaftserhaltung. In Dosierungen bis 500 µg/kg Körpergewicht waren die neuen Verbindungen der allgemeinen Formel I nicht oder nur sehr schwach gestagen wirksam, bei Erhöhung der täglichen Dosis auf 10 mg pro kg Körpergewicht war eine abgeschwächte gestagene Wirkung zu beobachten.

Insbesondere zeigen folgende Verbindungen eine besonders effektive pharmazeutische Wirkung:
5-[4-(5-Fluor-2-hydroxyphenyl)-2-hydroxy-4-methyl-2-trifluormethyl-valeroylamino]-phthalid
5-[4-(5-Fluor-2-hydroxyphenyl)-2-hydroxy-4-methyl-2-trifluormethyl-pentylamino]-phthalid
6-[4-(5-Fluor-2-methoxyphenyl)-2-hydroxy-4-methyl-2-trifluormethyl-valeroylamino]-4-methyl-2,3-benzoxazin-1-on
6-[4-(4-Brom-2-methoxyphenyl)-2-hydroxy-4-methyl-2-trifluormethyl-valeroylamino]-4-methyl-2,3-benzoxazin-1-on
6-[4-(5-Fluor-2-hydroxyphenyl)-2-hydroxy-4-methyl-2-trifluormethyl-valeroylamino]-4-methyl-2,3-benzoxazin-1-on

Aufgrund ihre anti - inflammatorischen und zusätzlichen antiallergischen, immunsuppressiven und anti - proliferativen Wirkung können die erfindungsgemäßen Verbindungen der allgemeinen Formel I als Medikamente zur Behandlung oder Prophylaxe folgender Krankheitszustände bei Säugetieren und Menschen Verwendung finden: Dabei steht der Begriff "ERKRANKUNG" für die folgenden Indikationen:
(i) Lungenerkrankungen
(ii) Rheumatische Erkrankungen / Autoimmunerkrankungen / Gelenkerkrankungen
(iii) Allergien
(iv) Gefäßentzündungen (Vaskulitiden)
(v) Dermatologische Erkrankungen
(vi) Nierenerkrankungen
(vii) Lebererkrankungen
(viii) Gastrointestinale Erkrankungen
(ix) Proktologische Erkrankungen
(x) Augenerkrankungen
(xi) Erkrankungen des Hals-Nasen-Ohren-Bereiches
(xii) Neurologische Erkrankungen
(xiii) Bluterkrankungen
(xiv) Tumorerkrankungen
(xv) Endokrine Erkrankungen
(xvi) Transplantationen
(xvii) Schwere Schockzustände
(xviii) Substitutionstherapie bei Nebenniereninsuffizienz
(xix) Emesis
(xx) Schmerzen bei entzündlicher Genese (z.B. Lumbago)

Darüber hinaus können die erfindungsgemäßen Verbindungen der allgemeinen Formel I zur Therapie und Prophylaxe weiterer oben nicht genannter Krankheitszustände eingesetzt werden, für die heute synthetische Glucocorticoide verwendet werden (siehe dazu Hatz, HJ, Glucocorticoide: Immunologische Grundlagen, Pharmakologie und Therapierichtlinien, Wissenschafliche Verlagsgesellschaft mbH, Stuttgart, 1998).

Für die therapeutische Wirkungen bei den oben genannten Krankheitszuständen ist die geeignete Dosis unterschiedlich und hängt beispielsweise von der Wirkstärke der Verbindung der allgemeinen Formel I, dem Wirt, der Art der Verabreichung und der Art und der Schwere der zu behandelnden Zustände, sowie der Verwendung als Prophylaktikum oder Therapeutikum ab.

## Patentansprüche

1. Verwendung mindestens einer Verbindung der allgemeinen Formel I zur Herstellung von Arzneimitteln mit entzündungshemmender Wirksamkeit 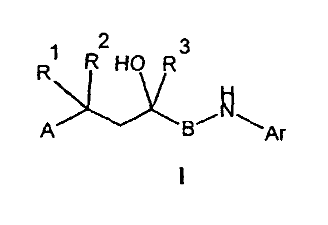 worin
R¹ und R² gleich oder verschieden sind und für ein Wasserstoffatom, eine C₁-C₅-Alkylgruppe oder, gemeinsam mit dem C-Atom der Kette, für einen Ring mit insgesamt 3-7 Gliedern,
R³ für eine C₁-C₅ Alkylgruppe oder eine teilweise oder vollständig fluorierte C₁-C₅ Alkylgruppe,
A Für die Gruppe 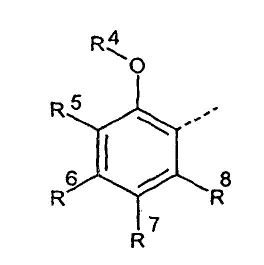 (die unterbrochene Linie bedeutet die Verknüpfungsstelle), worin R⁴ ein Wasserstoffatom, eine C₁-C₅-Alkylgruppe, eine C₁-C₁₀-Acylgruppe, eine C₃-C₁₀-Carbalkoxyalkylgruppe, eine C₂-C₅-Cyanalkylgruppe, eine C₃-C₁₀ unsubstituierte oder substituierte Allylgruppe, eine C₃-C₁₀ unsubstituierte oder substituierte Propargylgruppe, eine C₂-C₅-Alkoxyalkylgruppe, eine teilweise oder vollständig durch Fluoratome substituierte C₁-C₅-Alkylgruppe ,
R⁵ bis R⁸ gleich oder verschieden von einander und ausgewählt sind aus Wasserstoff- oder Halogenatomen oder C₁-C₅ Alkoxygruppen,
sowie R⁴ und R⁵ gemeinsam einen heterocyclischen Ring, der zusätzlich zum Sauerstoffatom gegebenenfalls mindestens ein weiteres Heteroatom aus der Gruppe Sauerstoff, Stickstoff, Schwefel enthalten kann, mit insgesamt 5-7 Gliedern bedeuten,
B Für eine Carbonyl- oder eine CH₂-Gruppe und
Ar Für ein Ringsystem, ausgewählt aus der Gruppe der allgemeinen Teilformeln **2** - **5**, stehen, 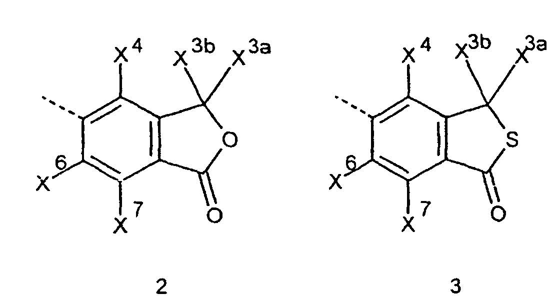 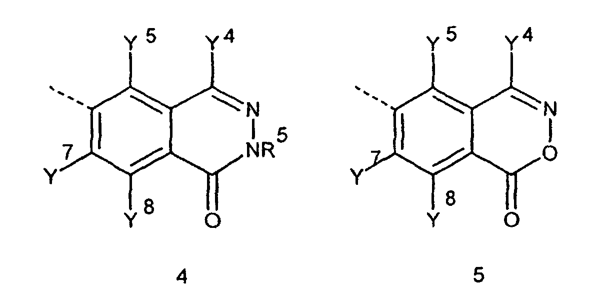
worin
die Reste X^{3a}, X^{3b}, X⁴, X⁶, X⁷ (in den Teilformeln **2** und **3**) und Y⁴, Y⁵, Y⁷, Y⁸ (in den Teilformeln **4** und **5**) gleich oder verschieden und ausgewählt sind aus Wasserstoffatomen, C₁-C₅-Alkylgruppen, teilweise oder vollständig fluorierten C₁-C₅ Alkylgruppen,
sowie darüber hinaus die Reste X⁴, X⁶, X⁷ (in den Teilformeln **2** und **3**) oder Y⁵, Y⁷, Y⁸ (in den Teilformeln **4** und **5**) ausgewählt sind aus Halogenatomen, Hydroxygruppen, C₁-C₅-Alkoxygruppen oder C₁-C₅-Alkanoyloxygruppen, sowie für den Fall, daß B für eine CH₂-Gruppe steht, die physiologisch verträglichen Salze der Verbindungen der allgemeinen Formel I mit Säuren.

2. Verwendung mindestens einer Verbindung der allgemeinen Formel I nach Anspruch 1 zur Herstellung von Arzneimitteln zur Behandlung mindestens einer der folgenden Erkrankungen:
(i) Lungenerkrankungen, die mit entzündlichen, allergischen und / oder proliferativen Prozessen einhergehen,
(ii) Rheumatische Erkrankungen / Autoimmunerkrankungen / Gelenkerkrankungen die mit entzündlichen, allergischen und / oder proliferativen Prozessen einhergehen,
(iii) Allergien, die mit entzündlichen, allergischen und / oder proliferativen Prozessen einhergehen,
(iv) Gefäßentzündungen (Vaskulitiden) die mit entzündlichen, allergischen und / oder proliferativen Prozessen einhergehen,
(v) Dermatologische Erkrankungen, die mit entzündlichen, allergischen und / oder proliferativen Prozessen einhergehen
(vi) Nierenerkrankungen, die mit entzündlichen, allergischen und / oder proliferativen Prozessen einhergehen
(vii) Lebererkrankungen, die mit entzündlichen, allergischen und / oder proliferativen Prozessen einhergehen
(viii) Gastrointestinale Erkrankungen, die mit entzündlichen, allergischen und / oder proliferativen Prozessen einhergehen:
(ix) Proktologische Erkrankungen, die mit entzündlichen, allergischen und / oder proliferativen Prozessen einhergehen,
(x) Augenerkrankungen, die mit entzündlichen, allergischen und / oder proliferativen Prozessen einhergehen,
(xi) Erkrankungen des Hals-Nasen-Ohren-Bereiches, die mit entzündlichen, allergischen und / oder proliferativen Prozessen einhergehen,
(xii) Neurologische Erkrankungen, die mit entzündlichen, allergischen und / oder proliferativen Prozessen einhergehen,
(xiii) Bluterkrankungen, die mit entzündlichen, allergischen und / oder proliferativen Prozessen einhergehen,
(xiv) Tumorerkrankungen, die mit entzündlichen, allergischen und / oder proliferativen Prozessen einhergehen,
(xv) Endokrine Erkrankungen, die mit entzündlichen, allergischen und / oder proliferativen Prozessen einhergehen,
(xvi) Transplantationen, die mit entzündlichen, allergischen und / oder proliferativen Prozessen einhergehen,
(xvii) Schwere Schockzustände, die mit entzündlichen, allergischen und / oder proliferativen Prozessen einhergehen,
(xviii) Substitutionstherapie bei Nebenniereninsuffizienz, die mit entzündlichen, allergischen und / oder proliferativen Prozessen einhergeht,
(xix) Emesis, die mit entzündlichen, allergischen und / oder proliferativen Prozessen einhergeht,
(xx) Schmerzen bei entzündlicher Genese (z.B. Lumbago), die mit entzündlichen, allergischen und / oder proliferativen Prozessen einhergehen.

3. Verwendung der Verbindungen der allgemeinen Formel I gemäß Anspruch 1 oder 2 in Form des Racemats oder in Form getrennt vorliegender Stereoisomere, wenn die Verbindung in unterschiedlichen Stereoisomeren vorliegen kann.

4. Verwendung nach Anspruch 1 oder 2, **dadurch gekennzeichnet, daß** eine Verbindung der allgemeinen Formel I verwendet wird, worin
R¹ und R² gleich oder verschieden sind und für ein Wasserstoffatom, eine Methyl- oder Ethylgruppe, oder gemeinsam mit dem C-Atom der Kette für einen Cyclopropylring stehen,
R³ für eine C₁-C₅ Perfluoralkylgruppe steht,
A die Gruppe 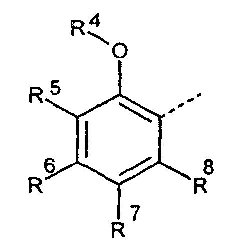 bedeutet, (die unterbrochene Linie bedeutet die Verknüpfungsstelle) worin
R⁴ für ein Wasserstoffatom, eine Methyl-, Ethyl-, Propyl- oder 2-Propylgruppe, eine Acetylgruppe, eine Methoxy-, Ethoxy- oder tert.Butoxycarbonylgruppe, eine Cyanmethyl-, 2-Cyanethylgruppe, eine Allylgruppe, eine Propargylgruppe, eine Methoxymethyl-, Methoxyethyl- oder Ethoxyethylgruppe, eine Mono-, Di- oder Trifluormethylgruppe, eine Pentafluorethyl- oder Nonafluorbutylgruppe steht,
R⁵ bis R⁸ in ein oder zwei Positionen Fluor- oder Chloratome und in den restlichen Positionen Wasserstoffatome bedeuten,
oder
R⁴ und R⁵ gemeinsam unter Einbeziehung der Phenyl-Ringatome 2 und 3 einen Furan-, einen Dihydrofuran- oder einen 2,3-Dihydro-1,4-dioxinring und R⁶, R⁷ und R⁸ Wasserstoffatome bedeuten,
X^{3a} für ein Wasserstoffatom oder eine Methylgruppe steht, oder
X^{3a} und X^{3b} gleich oder verschieden sind und für ein Wasserstoffatom oder eine Methylgruppe stehen,
X⁴, X⁶ und X⁷ gleich oder verschieden sind und unabhängig voneinander für ein Wasserstoffatom oder ein Fluor- oder ein Chloratom, und/oder
Y⁴ für eine Methyl- Ethyl-, Propyl-, 2-Propyl- oder Trifluormethylgruppe und/oder
Y⁵, Y⁷ und Y⁸ gleich oder verschieden sind und unabhängig voneinander für ein Wasserstoffatom oder ein Fluor- oder ein Chloratom
stehen,
und die anderen Substituenten die in der Formel I angegebenen Bedeutungen haben.

5. Verwendung nach Anspruch 1 oder 2, **dadurch gekennzeichnet, daß** eine Verbindung der allgemeinen Formel I verwendet wird, worin Ar für ein Ringsystem der Teilformel **2** oder **5** steht.

6. Verwendung mindestens einer Verbindung gemäß Anspruch 1 oder 2 ausgewählt aus
5-{2-Hydroxy-3-[1-(2-methoxyphenyl)-cyclopropyl]-2-trifluormethyl-propionylamino}-phthalid
5-{3-[1-(5-Fluor-2-methoxyphenyl)-cyclopropyl]-2-hydroxy-2-trifluormethyl-propionylamino}-phthalid
5-[2-Hydroxy-4-(2-methoxyphenyl)-4-methyl-2-trifluormethyl-valeroylamino]-phthalid
5-{3-[1-(5-Fluor-2-hydroxyphenyl)-cyclopropyl]-2-hydroxy-2-trifluormethyl-propionylamino}-phthalid
5-[4-(5-Fluor-2-hydroxyphenyl)-2-hydroxy-4-methyl-2-trifluormethyl-valeroylamino]-phthalid
5-[4-(5-Fluor-2-hydroxyphenyl)-2-hydroxy-4-methyl-2-trifluormethyl-pentylamino]-phthalid
4-Brom-5-[4-(5-fluor-2-hydroxyphenyl)-2-hydroxy-4-methyl-2-trifluormethyl-pentylamino]-phthalid
4-Brom-5-[4-(3-brom-5-fluor-2-hydroxyphenyl)-2-hydroxy-4-methyl-2-trifluormethyl-pentylamino]-phthalid
6-{3-[1-(5-Fluor-2-hydroxyphenyl)-cyclopropyl]-2-hydroxy-2-trifluormethyl-propionylamino}-4-methyl-2,3-benzoxazin-1-on
6-[4-(5-Fluor-2-methoxyphenyl)-2-hydroxy-4-methyl-2-trifluormethyl-valeroylamino]-4-methyl-2,3-benzoxazin-1-on
6-[4-(5-Fluor-2-hydroxyphenyl)-2-hydroxy-4-methyl-2-trifluormethyl-valeroylamino]-4-methyl-2,3-benzoxazin-1-on
6-[2-Hydroxy-4-(2-hydroxyphenyl)-4-methyl-2-trifluormethyl-valeroylamino]-4-ethyl-2,3-benzoxazin-1-on
6-[4-(5-Fluor-2-methoxyphenyl)-2-hydroxy-4-methyl-2-trifluormethyl-pentylamino]-4-methyl-2,3-benzoxazin-1-on
6-[4-(5-Fluor-2-hydroxyphenyl)-2-hydroxy-4-methyl-2-trifluormethyl-pentylamino]-4-methyl-2,3-benzoxazin-1-on
6-[4-(4-Brom-2-methoxyphenyl)-2-hydroxy-4-methyl-2-trifluormethyl-valeroylamino]-4-methyl-2,3-benzoxazin-1-on
6-[4-(4-Brom-2-methoxyphenyl)-2-hydroxy-4-methyl-2-trifluormethyl-pentylamino]-4-methyl-2,3-benzoxazin-1-on
und weiterhin alle Verbindungen aus den Tabellen 3-6 sowie den Beispielen 85 und 86.

7. Verbindungen der allgemeinen Formel I gemäß Anspruch 1, nämlich
5-{3-[1-(5-Fluor-2-methoxyphenyl)-cyclopropyl]-2-hydroxy-2-trifluormethyl-propionylamino}-phthalid
5-{3-[1-(5-Fluor-2-hydroxyphenyl)-cyclopropyl]-2-hydroxy-2-trifluormethyl-propionylamino}-phthalid
4-Brom-5-[4-(5-fluor-2-hydroxyphenyl)-2-hydroxy-4-methyl-2-trifluormethyl-pentylamino]-phthalid
4-Brom-5-[4-(3-brom-5-fluor-2-hydroxyphenyl)-2-hydroxy-4-methyl-2-trifluormethyl-pentylamino]-phthalid
6-{3-[1-(5-Fluor-2-hydroxyphenyl)-cyclopropyl]-2-hydroxy-2-trifluormethyl-propionylamino}-4-methyl-2,3-benzoxazin-1-on
6-[2-Hydroxy-4-(2-hydroxyphenyl)-4-methyl-2-trifluormethyl-valeroylamino]-4-ethyl-2,3-benzoxazin-1-on
6-[4-(4-Brom-2-methoxyphenyl)-2-hydroxy-4-methyl-2-trifluormethyl-pentylamino]-4-methyl-2,3-benzoxazin-1-on
5-{3-[1-(5-Fluor-2-methoxyphenyl)-cyclopropyl]-2-hydroxy-2-trifluormethyl-propionylamino}-phthalid
6-[2-Hydroxy-4-(5-fluor-2-methoxyphenyl)-4-methyl-2-trifluormethyl-pentylamino]-4-methyl-2,3-benzoxazin-1-on
5-{3-[1-(5-Fluor-2-hydroxyphenyl)-cyclopropyl]-2-hydroxy-2-trifluormethyl-propionylamino}-phthalid
6-{3-[1-(5-Fluor-2-hydroxyphenyl)-cyclopropyl]-2-hydroxy-2-trifluormethyl-propionylamino}-4-methyl-2,3-benzoxazin-1-on
(-)-4-Brom-5-[4-(5-fluor-2-hydroxyphenyl)-2-hydroxy-4-methyl-2-trifluormethyl-pentylamino]-phthalid
(-)-4-Brom-5-[4-(3-brom-5-fluor-2-hydroxyphenyl)-2-hydroxy-4-methyl-2-trifluormethyl-pentylamino]-phthalid
5-[2-Hydroxy-4-(5-isopropyl-2-methoxyphenyl)-4-methyl-2-trifluormethyl-valeroylamino]-phthalid
5-[2-Hydroxy-4-(2-methoxy-5-propyl-phenyl)-4-methyl-2-trifluormethyl-valeroylamino]-phthalid
5-[2-Hydroxy-4-(2-benzyloxy-5-fluorphenyl)-4-methyl-2-trifluormethyl-valeroylamino]-phthalid
5-[2-Hydroxy-4-(2-difluormethoxy-5-fluorphenyl)-4-methyl-2-trifluormethyl-valeroylamino]-phthalid
5-[2-Hydroxy-4-(5-fluor-2-methoxymethoxy-phenyl)-4-methyl-2-trifluormethyl-valeroylamino]-phthalid
5-[2-Hydroxy-4-(2-ethoxymethoxy-5-fluorphenyl)-4-methyl-2-trifluormethyl-valeroylamino]-phthalid
5-{[2-Hydroxy-4-[5-fluor-2-(2-methoxyethoxy)-phenyl]-4-methyl-2-trifluormethyl-valeroylamino}-phthalid

8. Pharmazeutische Präparate, enthaltend mindestens eine Verbindung gemäß Anspruch 7 sowie einen pharmazeutisch verträglichen Träger.

9. Verwendung mindestens einer Verbindung gemäß Anspruch 7 zur Herstellung von Arzneimitteln.

## Claims

1. Use of at least one compound of general formula I for the production of pharmaceutical agents that have an anti-inflammatory activity 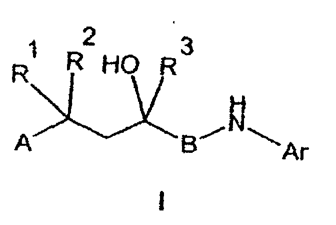 in which
R¹ and R² are the same or different and stand for a hydrogen atom, a C₁-C₅ alkyl group, or, together with the C-atom of the chain, stand for a ring with a total of 3-7 members,
R³ stands for a C₁-C₅ alkyl group or a partially or completely fluorinated C₁-C₅ alkyl group,
A stands for the group 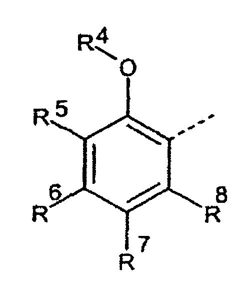 (the dashed line denotes the interface site), in which R⁴ means a hydrogen atom, a C₁-C₅ alkyl group, a C₁-C₁₀ acyl group, a C₃-C₁₀ carbalkoxyalkyl group, a C₂-C₅ cyanoalkyl group, a C₃-C₁₀ unsubstituted or substituted allyl group, a C₃-C₁₀ unsubstituted or substituted propargyl group, a C₂-C₅ alkoxyalkyl group, a C₁-C₅ alkyl group that is partially or completely substituted by fluorine atoms,
R⁵ to R⁸ are the same or different from one another and are selected from hydrogen or halogen atoms or C₁-C₅ alkoxy groups,
and R⁴ and R⁵ together denote a heterocyclic ring, which in addition to the oxygen atom optionally can contain at least one other heteroatom from the group of oxygen, nitrogen, sulphur, with a total of 5-7 members,
B stands for a carbonyl group or a CH₂ group, and
Ar stands for a ring system, selected from the group of general partial formulas 2-5, 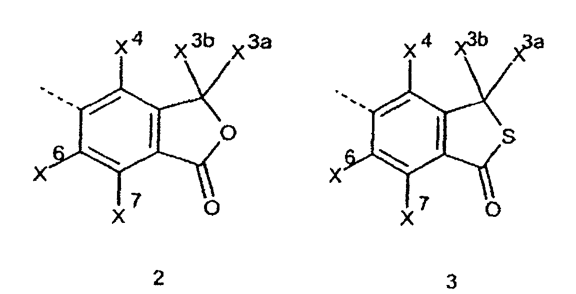 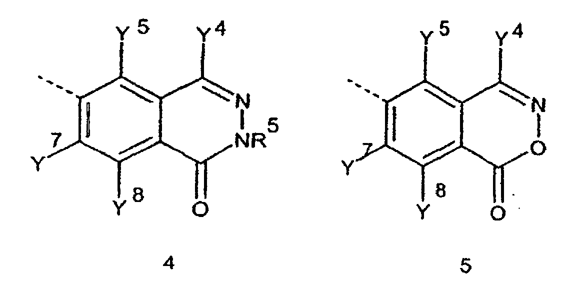
in which
radicals X^{3a}, X^{3b}, X⁴, X⁶, X⁷ (in partial formulas 2 and 3) and Y⁴, Y⁵, Y⁷, Y⁸ (in partial formulas 4 and 5) are the same or different and are selected from hydrogen atoms, C₁-C₅ alkyl groups, partially or completely fluorinated C₁-C₅ alkyl groups,
and, moreover, radicals X⁴, X⁶, X⁷ (in partial formulas 2 and 3) or Y⁵, Y⁷, Y⁸ (in partial formulas 4 and 5) are selected from halogen atoms, hydroxy groups, C₁-C₅ alkoxy groups or C₁-C₅ alkanoyloxy groups, and, if B stands for a CH₂ group, the physiologically compatible salts of the compounds of general formula I with acids.

2. Use of at least one compound of general formula I according to Claim 1 for the production of pharmaceutical agents for treatment of at least one of the following diseases:
(i) Lung diseases which coincide with inflammatory, allergic and/or proliferative processes,
(ii) Rheumatic diseases/autoimmune diseases/joint diseases which coincide with inflammatory, allergic and/or proliferative processes,
(iii) Allergies which coincide with inflammatory, allergic and/or proliferative processes,
(iv) Vascular inflammations (vasculitides) which coincide with inflammatory and/or proliferative processes,
(v) Dermatological diseases which coincide with inflammatory, allergic and/or proliferative processes,
(vi) Kidney diseases which coincide with inflammatory, allergic and/or proliferative processes,
(vii) Liver diseases which coincide with inflammatory, allergic and/or proliferative processes,
(viii) Gastrointestinal diseases which coincide with inflammatory, allergic and/or proliferative processes,
(ix) Proctological diseases which coincide with inflammatory, allergic and/or proliferative processes,
(x) Eye diseases which coincide with inflammatory, allergic and/or proliferative processes,
(xi) Diseases of the ear-nose-throat area which coincide with inflammatory, allergic and/or proliferative processes,
(xii) Neurological diseases, which coincide with inflammatory, allergic and/or proliferative processes,
(xiii) Blood diseases which coincide with inflammatory, allergic and/or proliferative processes,
(xiv) Tumour diseases which coincide with inflammatory, allergic and/or proliferative processes,
(xv) Endocrine diseases which coincide with inflammatory, allergic and/or proliferative processes,
(xvi) Transplants which coincide with inflammatory, allergic and/or proliferative processes,
(xvii) Severe shock conditions which coincide with inflammatory, allergic and/or proliferative processes,
(xviii) Substitution therapy in the case of suprarenal insufficiency which coincides with inflammatory, allergic and/or proliferative processes,
(xix) Emesis which coincides with inflammatory, allergic and/or proliferative processes,
(xx) Pain with inflammatory origin, e.g., lumbago which coincides with inflammatory, allergic and/or proliferative processes.

3. Use of the compounds of general formula I according to Claim 1 or 2 in the form of the racemate or in the form of separately present stereoisomers, if the compound is able to exist in different stereoisomers.

4. Use according to Claim 1 or 2, **characterized in that** a compound of general formula I is used in which
R¹ and R² are the same or different and stand for a hydrogen atom, a methyl or ethyl group; or together with the C atom of the chain, R¹ and R² stand for a cyclopropyl ring,
R³ stands for a C₁-C₅ perfluoroalkyl group,
A stands for the group 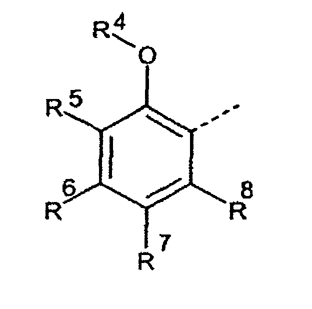 (the dashed line denotes the interface site), in which
R⁴ means a hydrogen atom, a methyl, ethyl, propyl or 2-propyl group, an acetyl group, a methoxy, ethoxy or tert-butoxycarbonyl group, a cyanomethyl group, a 2-cyanoethyl group, an allyl group, a propargyl group, a methoxymethyl, methoxyethyl or ethoxyethyl group, a mono-, di- or trifluoromethyl group, a pentafluoroethyl or nonafluorobutyl group,
R⁵ to R⁸ in one or two positions mean fluorine or chlorine atoms and in the remaining positions mean hydrogen atoms,
or
R⁴ and R⁵ together with incorporation of phenyl-ring atoms 2 and 3 mean a furan, a dihydrofuran or a 2,3-dihydro-1,4-dioxine ring and R⁶, R⁷ and
R⁸ mean hydrogen atoms,
X³ₐ stands for a hydrogen atom or a methyl group, or
X^{3a} and X^{3b} are the same or different and stand for a hydrogen atom or a methyl group,
X⁴, X⁶ and X⁷ are the same or different and, independently of one another, stand for a hydrogen atom or a fluorine or a chlorine atom, and/or
Y⁴ stands for a methyl, ethyl, propyl, 2-propyl or trifluoromethyl group and/or
Y⁵, Y⁷ and Y⁸ are the same or different and, independently of one another, stand for a hydrogen atom or a fluorine atom or a chlorine atom,
and the other substituents have the meanings that are indicated in formula I.

5. Use according to Claim 1 or 2, **characterized in that** a compound of general formula I is used in which Ar stands for a ring system of partial formula 2 or 5.

6. Use of at least one compound according to Claim 1 or 2 selected from
5-{2-hydroxy-3-[1-(2-methoxyphenyl)-cyclopropyl]-2-trifluoromethyl-propionylamino}-phthalide
5-{3-[1-(5-fluoro-2-methoxyphenyl)-cyclopropyl]-2-hydroxy-2-trifluoromethyl-propionylamino}-phthalide
5-[2-hydroxy-4-(2-methoxyphenyl)-4-methyl-2-trifluoromethyl-valeroylamino]-phthalide
5-{3-[1-(5-fluoro-2-hydroxyphenyl)-cyclopropyl]-2-hydroxy-2-trifluoromethyl-propionylamino}-phthalide
5-[4-(5-fluoro-2-hydroxyphenyl)-2-hydroxy-4-methyl-2-trifluoromethyl-valeroylamino]-phthalide
5-[4-(5-fluoro-2-hydroxyphenyl)-2-hydroxy-4-methyl-2-trifluoromethyl-pentylamino]-phthalide
4-bromo-5-[4-(5-fluoro-2-hydroxyphenyl)-2-hydroxy-4-methyl-2-trifluoromethyl-pentylamino]-phthalide
4-bromo-5-[4-(3-bromo-5-fluoro-2-hydroxyphenyl)-2-hydroxy-4-methyl-2-trifluoromethyl-pentylamino]-phthalide
6-{3-[1-(5-fluoro-2-hydroxyphenyl)-cyclopropyl]-2-hydroxy-2-trifluoromethyl-propionylamino}-4-methyl-2,3-benzoxazin-1-one
6-[4-(5-fluoro-2-methoxyphenyl)-2-hydroxy-4-methyl-2-trifluoromethyl-valeroylamino]-4-methyl-2,3-benzoxazin-1-one
6-[4-(5-fluoro-2-hydroxyphenyl)-2-hydroxy-4-methyl-2-trifluoromethyl-valeroylamino]-4-methyl-2,3-benzoxazin-1-one
6-[2-hydroxy-4-(2-hydroxyphenyl)-4-methyl-2-trifluoromethyl-valeroylamino]-4-ethyl-2,3-benzoxazin-1-one
6-[4-(5-fluoro-2-methoxyphenyl)-2-hydroxy-4-methyl-2-trifluoromethyl-pentylamino]-4-methyl-2,3-benzoxazin-1-one
6-[4-(5-fluoro-2-hydroxyphenyl)-2-hydroxy-4-methyl-2-trifluoromethyl-pentylamino]-4-methyl-2,3-benzoxazin-1-one
6-[4-(4-bromo-2-methoxyphenyl)-2-hydroxy-4-methyl-2-trifluoromethyl-valeroylamino]-4-methyl-2,3-benzoxazin-1-one
6-[4-(4-bromo-2-methoxyphenyl)-2-hydroxy-4-methyl-2-trifluoromethyl-pentylamino]-4-methyl-2,3-benzoxazin-1-one
and also all compounds from Tables 3-6 and Examples 85 and 86.

7. Compounds of general formula I according to Claim 1, namely
5-{3-[1-(5-fluoro-2-methoxyphenyl)-cyclopropyl]-2-hydroxy-2-trifluoromethyl-propionylamino}-phthalide
5-{3-[1-(5-fluoro-2-hydroxyphenyl)-cyclopropyl]-2-hydroxy-2-trifluoromethyl-propionylamino}-phthalide
4-bromo-5-[4-(5-fluoro-2-hydroxyphenyl)-2-hydroxy-4-methyl-2-trifluoromethyl-pentylamino]-phthalide
4-bromo-5-[4-(3-bromo-5-fluoro-2-hydroxyphenyl)-2-hydroxy-4-methyl-2-trifluoromethyl-pentylamino]-phthalide
6-{3-[1-(5-fluoro-2-hydroxyphenyl)-cyclopropyl]-2-hydroxy-2-trifluoromethyl-propionylamino}-4-methyl-2,3-benzoxazin-1-one
6-[2-hydroxy-4-(2-hydroxyphenyl)-4-methyl-2-trifluoromethyl-valeroylamino]-4-ethyl-2,3-benzoxazin-1-one
6-[4-(4-bromo-2-methoxyphenyl)-2-hydroxy-4-methyl-2-trifluoromethyl-pentylamino]-4-methyl-2,3-benzoxazin-1-one
5-{3-[1-(5-fluoro-2-methoxyphenyl)-cyclopropyl]-2-hydroxy-2-trifluoromethyl-propionylamino}-phthalide
6-[2-hydroxy-4-(5-fluoro-2-methoxyphenyl)-4-methyl-2-trifluoromethyl-pentylamino]-4-methyl-2,3-benzoxazin-1-one
5-{3-[1-(5-fluoro-2-hydroxyphenyl)-cyclopropyl]-2-hydroxy-2-trifluoromethyl-propionylamino}-phthalide
6-{3-[1-(5-fluoro-2-hydroxyphenyl)-cyclopropyl]-2-hydroxy-2-trifluoromethyl-propionylamino}-4-methyl-2,3-benzoxazin-1-one
(-)-4-bromo-5-[4-(5-fluoro-2-hydroxyphenyl)-2-hydroxy-4-methyl-2-trifluoromethyl-pentylamino]-phthalide
(-)-4-bromo-5-[4-(3-bromo-5-fluoro-2-hydroxy-phenyl)-2-hydroxy-4-methyl-2-trifluoromethyl-pentylamino]-phthalide
5-[2-hydroxy-4-(5-isopropyl-2-methoxyphenyl)-4-methyl-2-trifluoromethyl-valeroylamino]-phthalide
5-[2-hydroxy-4-(2-methoxy-5-propyl-phenyl)-4-methyl-2-trifluoromethyl-valeroylamino]-phthalide
5-[2-hydroxy-4-(2-benzyloxy-5-fluorophenyl)-4-methyl-2-trifluoromethyl-valeroylamino]-phthalide
5-[2-hydroxy-4-(2-difluoromethoxy-5-fluorophenyl)-4-methyl-2-trifluoromethyl-valeroylamino]-phthalide
5-[2-hydroxy-4-(5-fluoro-2-methoxymethoxy-phenyl)-4-methyl-2-trifluoromethyl-valeroylamino]-phthalide
5-[2-hydroxy-4-(2-ethoxymethoxy-5-fluorophenyl)-4-methyl-2-trifluoromethyl-valeroylamino]-phthalide
5-{[2-hydroxy-4-[5-fluoro-2-(2-methoxyethoxy)-phenyl]-4-methyl-2-trifluoromethyl-valeroylamino}-phthalide

8. Pharmaceutical products comprising at least one compound according to Claim 7 and also a pharmaceutically compatible vehicle.

9. Use of at least one compound according to Claim 7 for the production of pharmaceutical agents.

## Revendications

1. Utilisation d'au moins un composé de formule générale I pour la fabrication de médicaments à activité anti-inflammatoire 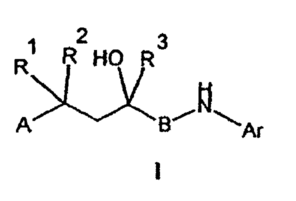 formule dans laquelle
R¹ et R² sont identiques ou différents et représentent un atome d'hydrogène, un groupe alkyle en C₁-C₅ ou, conjointement avec l'atome de carbone de la chaîne, un cycle ayant au total 3-7 chaînons,
R³ représente un groupe alkyle en C₁-C₅ ou un groupe alkyle en C₁-C₅ partiellement ou totalement fluoré,
A représente le groupe 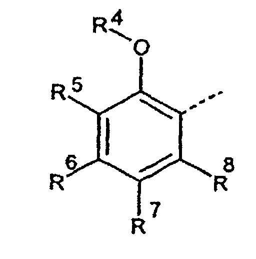 (le trait interrompu représente la position de liaison), dans lequel R⁴ représente un atome d'hydrogène, un groupe alkyle en C₁-C₅, un groupe acyle en C₁-C₁₀, un groupe carbalcoxyalkyle en C₃-C₁₀, un groupe cyanoalkyle en C₂-C₅, un groupe allyle en C₃-C₁₀, substitué ou non substitué, un groupe propargyle en C₃-C₁₀ substitué ou non substitué, un groupe alcoxyalkyle en C₂-C₅, un groupe alkyle en C₁-C₅ partiellement ou totalement substitué par des atomes de fluor,
R⁵ à R⁸ sont identiques ou différents l'un de l'autre et sont choisis parmi des atomes d'hydrogène et d'halogène et des groupes alcoxy en C₁-C₅,
et R⁴ et R⁵ peuvent former ensemble un cycle hétérocyclique qui, en plus de l'atome d'oxygène, peut éventuellement comporter au moins un autre hétéroatome choisi parmi les atomes d'oxygène, d'azote et de soufre, et ayant au total 5-7 chaînons,
B représente un groupe carbonyle ou un groupe CH₂ et
Ar représente un système cyclique choisi dans l'ensemble des formules partielles générales **2-5**, 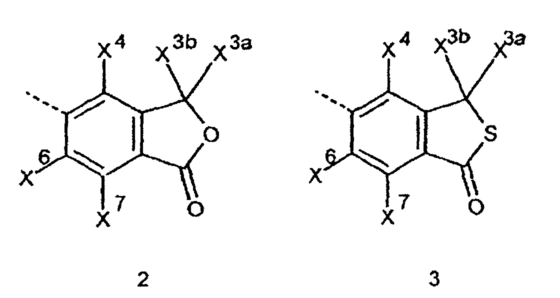 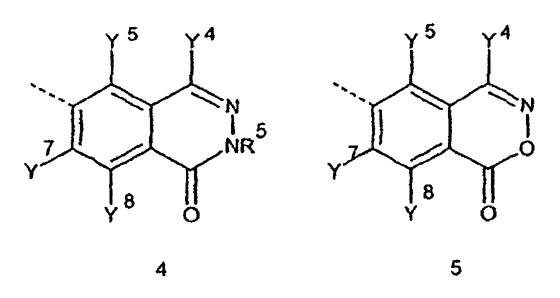
dans lesquelles
les radicaux X^{3a}, X^{3b}, X⁴, X⁶, X⁷ (dans les formules partielles **2** et **3**) et Y⁴, Y⁵, Y⁷, Y⁸ (dans les formules partielles **4** et **5**) sont identiques ou différents et sont choisis parmi des atomes d'hydrogène, des groupes alkyle en C₁-C₅, des groupes alkyle en C₁-C₅ partiellement ou totalement fluorés,
et en outre les radicaux X⁴, X⁶, X⁷ (dans les formules partielles **2** et **3**) ou Y⁵, Y⁷, Y⁸ (dans les formules partielles **4** et **5**) sont choisis parmi des atomes d'halogène, des groupes hydroxy, des groupes alcoxy en C₁-C₅ et des groupes alcanoyloxy en C₁-C₅, et dans le cas où B représente un groupe CH₂, des sels physiologiquement acceptables des composés de formule générale I avec des acides.

2. Utilisation d'au moins un composé de formule générale I selon la revendication 1, pour la fabrication de médicaments destinés au traitement d'au moins l'une des maladies suivantes :
(i) des maladies pulmonaires qui s'accompagnent de processus inflammatoires, allergiques et/ou prolifératifs,
(ii) des maladies rhumatismales/des maladies auto-immunes/des maladies des articulations, qui s'accompagnent de processus inflammatoires, allergiques et/ou prolifératifs,
(iii) des allergies qui s'accompagnent de processus inflammatoires, allergiques et/ou prolifératifs,
(iv) des inflammations vasculaires (angéites) qui s'accompagnent de processus inflammatoires, allergiques et/ou prolifératifs,
(v) des maladies dermatologiques qui s'accompagnent de processus inflammatoires, allergiques et/ou prolifératifs,
(vi) des néphropathies qui s'accompagnent de processus inflammatoires, allergiques et/ou prolifératifs,
(vii) des hépatopathies qui s'accompagnent de processus inflammatoires, allergiques et/ou prolifératifs,
(viii) des maladies gastro-intestinales qui s'accompagnent de processus inflammatoires, allergiques et/ou prolifératifs,
(ix) des maladies proctologiques qui s'accompagnent de processus inflammatoires, allergiques et/ou prolifératifs,
(x) des maladies des yeux qui s'accompagnent de processus inflammatoires, allergiques et/ou prolifératifs,
(xi) des maladies du domaine oto-rhino-laryngologique qui s'accompagnent de processus inflammatoires, allergiques et/ou prolifératifs,
(xii) des maladies neurologiques qui s'accompagnent de processus inflammatoires, allergiques et/ou prolifératifs,
(xiii) des maladies du sang qui s'accompagnent de processus inflammatoires, allergiques et/ou prolifératifs,
(xiv) des maladies tumorales qui s'accompagnent de processus inflammatoires, allergiques et/ou prolifératifs,
(xv) des maladies endocriniennes qui s'accompagnent de processus inflammatoires, allergiques et/ou prolifératifs,
(xvi) des transplantations qui s'accompagnent de processus inflammatoires, allergiques et/ou prolifératifs,
(xvii) des états de choc sérieux qui s'accompagnent de processus inflammatoires, allergiques et/ou prolifératifs,
(xviii) la thérapie de remplacement, dans le cas d'insuffisance surrénale, qui s'accompagne de processus inflammatoires, allergiques et/ou prolifératifs,
(xix) des vomissements qui s'accompagnent de processus inflammatoires, allergiques et/ou prolifératifs,
(xx) des douleurs d'origine inflammatoire (par exemple le lombago) qui s'accompagnent de processus inflammatoires, allergiques et/ou prolifératifs.

3. Utilisation des composés de formule générale I selon la revendication 1 ou 2, sous forme du racémate ou sous forme de stéréo-isomères présents séparément, lorsque le composé peut se trouver sous forme de stéréo-isomères différents.

4. Utilisation selon la revendication 1 ou 2, **caractérisée en ce qu'**on utilise un composé de formule générale I dans laquelle
R¹ et R² sont identiques ou différents et représentent un atome d'hydrogène, un groupe méthyle ou éthyle, ou forment ensemble avec l'atome de carbone de la chaîne un cycle cyclopropyle,
R³ représente un groupe perfluoroalkyle en C₁-C₅,
A représente le groupe 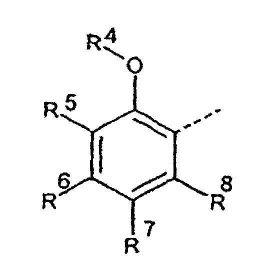 (le trait interrompu représente la position de liaison), dans lequel
R⁴ représente un atome d'hydrogène, un groupe méthyle, éthyle, propyle ou 2-propyle, un groupe acétyle, un groupe méthoxy, éthoxy ou tert-butoxycarbonyle, un groupe cyanométhyle, 2-cyanoéthyle, un groupe allyle, un groupe propargyle, un groupe méthoxyméthyle, méthoxyéthyle ou éthoxyéthyle, un groupe mono-, di- ou trifluorométhyle, un groupe pentafluoroéthyle ou nonafluorobutyle,
R⁵ à R⁸ représentent en une ou deux positions des atomes de fluor ou de chlore et dans les positions restantes des atomes d'hydrogène,
ou
R⁴ et R⁵ forment ensemble, avec inclusion des atomes 2 et 3 formant le cycle phényle, un cycle furanne, un cycle dihydrofuranne ou un cycle 2,3-dihydro-1,4-dioxine, et R⁶, R⁷ et R⁸ représentent des atomes d'hydrogène,
X^{3a} représente un atome d'hydrogène ou un groupe méthyle, ou
X^{3a} et X^{3b} sont identiques ou différents et représentent un atome d'hydrogène ou un groupe méthyle,
X⁴, X⁶ et X⁷ sont identiques ou différents et représentent, indépendamment les uns des autres, un atome d'hydrogène ou un atome de fluor ou un atome de chlore, et/ou
Y⁴ représente un groupe méthyle, éthyle, propyle, 2-propyle ou trifluorométhyle, et/ou
Y⁵, Y⁷ et Y⁸ sont identiques ou différents et représentent, indépendamment les uns des autres, un atome d'hydrogène ou un atome de fluor ou un atome de chlore,
et les autres substituants ont les significations indiquées dans la formule I.

5. Utilisation selon la revendication 1 ou 2, **caractérisée en ce qu'**on utilise un composé de formule générale I dans laquelle Ar représente un système cyclique de la formule partielle **2** ou **5**.

6. Utilisation d'au moins un composé selon la revendication 1 ou 2, choisi parmi les composés suivants
5-{2-hydroxy-3-[2-(2-méthoxyphényl)cyclopropyl]-2-trifluorométhyl-propionylamino}phtalide
5-{3-[1-(5-fluoro-2-méthoxyphényl)cyclopropyl]-2-hydroxy-2-trifluorométhyl-propionylamino}phtalide
5-[2-hydroxy-4-(2-méthoxyphényl)-4-méthyl-2-trifluorométhyl-valéroylamino]phtalide
5-{3-[1-(5-fluoro-2-hydroxyphényl)cyclopropyl]-2-hydroxy-2-trifluorométhyl-propionylamino}phtalide
5-[4-(5-fluoro-2-hydroxyphényl)-2-hydroxy-4-méthyl-2-trifluorométhyl-valéroylamino]phtalide
5-[4-(5-fluoro-2-hydroxyphényl)-2-hydroxy-4-méthyl-2-trifluorométhyl-pentylamino]phtalide
4-bromo-5-[4-(5-fluoro-2-hydroxyphényl)-2-hydroxy-4-méthyl-2-trifluorométhyl-pentylamino]phtalide
4-bromo-5-[4-(3-bromo-5-fluoro-2-hydroxyphényl)-2-hydroxy-4-méthyl-2-trifluorométhyl-pentylamino]phtalide
6-{3-[1-(5-fluoro-2-hydroxyphényl)cyclopropyl]-2-hydroxy-2-trifluorométhyl-propionylamino}-4-méthyl-2,3-benzoxazin-1-one
6-[4-(5-fluoro-2-méthoxyphényl)-2-hydroxy-4-méthyl-2-trifluorométhyl-valéroylamino]-4-méthyl-2,3-benzoxazin-1-one
6-[4-(5-fluoro-2-hydroxyphényl)-2-hydroxy-4-méthyl-2-trifluorométhyl-valéroylamino]-4-méthyl-2,3-benzoxazin-1-one
6-[2-hydroxy-4-(2-hydroxyphényl)-4-méthyl-2-trifluorométhyl-valéroylamino]-4-éthyl-2,3-benzoxazin-1-one
6-[4-(5-fluoro-2-méthoxyphényl)-2-hydroxy-4-méthyl-2-trifluorométhyl-pentylamino]-4-méthyl-2,3-benzoxazin-1-one
6-[4-(5-fluoro-2-hydroxyphényl)-2-hydroxy-4-méthyl-2-trifluorométhyl-pentylamino]-4-méthyl-2,3-benzoxazin-1-one
6-[4-(4-bromo-2-méthoxyphényl)-2-hydroxy-4-méthyl-2-trifluorométhyl-valéroylamino]-4-méthyl-2,3-benzoxazin-1-one
6-[4-(4-bromo-2-méthoxyphényl)-2-hydroxy-4-méthyl-2-trifluorométhyl-pentylamino]-4-méthyl-2,3-benzoxazin-1-one
et en outre tous les composés des tableaux 3-6 ainsi que des exemples 85 et 86.

7. Composés de formule générale I selon la revendication 1, à savoir
5-{3-[1-(5-fluoro-2-méthoxyphényl)cyclopropyl]-2-hydroxy-2-trifluorométhyl-propionylamino}phtalide
5-{3-[1-(5-fluoro-2-hydroxyphényl)cyclopropyl]-2-hydroxy-2-trifluorométhyl-propionylamino}phtalide
4-bromo-5-[4-(5-fluoro-2-hydroxyphényl)-2-hydroxy-4-méthyl-2-trifluorométhyl-pentylamino}phtalide
4-bromo-5-[4-(3-bromo-5-fluoro-2-hydroxyphényl)-2-hydroxy-4-méthyl-2-trifluorométhyl-pentylamino]phtalide
6-{3-[1-(5-fluoro-2-hydroxyphényl)cyclopropyl]-2-hydroxy-2-trifluorométhyl-propionylamino}-4-méthyl-2,3-benzoxazin-1-one
6-[2-hydroxy-4-(2-hydroxyphényl)-4-méthyl-2-trifluorométhyl-valéroylamino]-4-éthyl-2,3-benzoxazin-1-one
6-[4-(4-bromo-2-méthoxyphényl)-2-hydroxy-4-méthyl-2-trifluorométhyl-pentylamino]-4-méthyl-2,3-benzoxazin-1-one
5-{3-[1-(5-fluoro-2-méthoxyphényl)cyclopropyl]-2-hydroxy-2-trifluorométhyl-propionylamino}phtalide
6-[2-hydroxy-4-(5-fluoro-2-méthoxyphényl)-4-méthyl-2-trifluorométhyl-pentylamino]-4-méthyl-2,3-benzoxazin-1-one
5-{3-[1-(5-fluoro-2-hydroxyphényl)cyclopropyl]-2-hydroxy-2-trifluorométhyl-propionylamino}phtalide
6-{3-[1-(5-fluoro-2-hydroxyphényl)cyclopropyl]-2-hydroxy-2-trifluorométhyl-propionylamino}-4-méthyl-2,3-benzoxazin-1-one
(-)-4-bromo-5-[4-(5-fluoro-2-hydroxyphényl)-2-hydroxy-4-méthyl-2-trifluorométhyl-pentylamino]phtalide
(-)-4-bromo-5-[4-(3-bromo-5-fluoro-2-hydroxyphényl)-2-hydroxy-4-méthyl-2-trifluorométhyl-pentylamino]phtalide
5-[2-hydroxy-4-(5-isopropyl-2-méthoxyphényl)-4-méthyl-2-trifluorométhyl-valéroylamino]phtalide
5-[2-hydroxy-4-(2-méthoxy-5-propylphényl)-4-méthyl-2-trifluorométhyl-valéroylamino]phtalide
5-[2-hydroxy-4-(2-benzyloxy-5-fluorophényl)-4-méthyl-2-trifluorométhyl-valéroylamino]phtalide
5-[2-hydroxy-4-(2-difluorométhoxy-5-fluorophényl)-4-méthyl-2-trifluorométhyl-valéroylamino]phtalide
5-[2-hydroxy-4-(5-fluoro-2-méthoxyméthoxyphényl)-4-méthyl-2-trifluorométhyl-valéroylamino]phtalide
5-[2-hydroxy-4-(2-éthoxyméthoxy-5-fluorophényl)-4-méthyl-2-trifluorométhyl-valéroylamino]phtalide
5-{[2-hydroxy-4-[5-fluoro-2-(2-méthoxyéthoxy)phényl]-4-méthyl-2-trifluorométhyl-valéroylamino}phtalide.

8. Préparations pharmaceutiques contenant au moins un composé selon la revendication 7 ainsi qu'un véhicule pharmaceutiquement acceptable.

9. Utilisation d'au moins un composé selon la revendication 7, pour la fabrication de médicaments.
